# EUROPEAN PATENT APPLICATION

(11) **EP 4 506 018 A1**
(43) Date of publication of application: **12.02.2025**
(21) Application number: 23784991.4
(22) Date of filing: 05.04.2023
(51) Int. Cl.: A61K 47/68, A61K 38/50, A61P 35/00

(54) **NOVEL TUMOR ANTIGEN-TARGETING ANTICANCER AGENT**

(30) Priority: 05.04.2022 KR 20220042556
(71) Applicant: Industry Foundation of Chonnam National University, Gwangju 61186 (KR)
(72) Inventor: HONG, Yeongjin, Gwangju 61682 (KR); MIN, Jung-Joon, Gwangju 61461 (KR); YOU, Sung-Hwan, Gwangju 61419 (KR); ZHANG, Ying, Hwasun-gun Jeollanam-do 58116 (KR); RUKHSORA, Sultonova, Hwasun-gun Jeollanam-do 58116 (KR)
(74) Representative: Dehns
(86) International application number: PCT/KR2023/004590
(87) International publication number: WO 2023/195767

(57) **Abstract**

The present invention relates to a novel fusion protein in which a monobody specifically bidning to a tumor antigen is linked to L-asparaginase for treating solid cancer more efficiently, and a use thereof, paticularly to a novel fusion protein comprising a recombinant monobody which specifically binds to clareticulin and an L-asparaginase linked to the C-terminus of the recombinant monobody, wherein the recombinant monobody has a peptide that specifically binds to the calreticulin inserted into at least one of the BC loop and the FG loop of human fibronectin domain III (Fn3) as an asparaginase-based novel anticancer agent targeting tumor antigen whose drug delivery efficacy to tumor tissue is enhanced.

## Description

### TECHNICAL FIELD

The present invention relates to a novel anticancer agent, and more particularly to a novel anticancer agent targeting tumor antigens.

### BACKGROUND ART

Tumor antigens are molecules that are specifically expressed on cancer cells or are overexpressed compared to normal cells. Tumor antigens are often closely associated with the proliferation and metastasis of cancer cells, therefore, administration of molecules such as antibodies that can block the function of these tumor antigens can inhibit the growth or metastasis of tumor tissue. Targeted anticancer agents are being developed using the tumor antigen-specific binding capability of the molecules such as the antibodies that can selectively bind to tumor antigens. Those targeted anticancer agents can selectively attack tumor cells by linking themselves or therapeutic radionuclides to tumor antigen-targeting molecules through a covalent bond or a non-covalent bond such as a coordination bond with chelators. In addition, in recent years, a therapeutic strategy has been adopted in which a chimeric antigen receptor (CAR), a fusion protein prepared by linking an antibody fragment specific for a tumor antigen (e.g., scFv) to the intracellular signal transduction domain of a TCR, is transfected into innate immune cells such as T cells or NK cells to enhance their innate immunity, and the cells are then used as anticancer therapeutics. For example, European Patent Publication EP12102374B1 discloses the use of a monobody that specifically binds to PMSA and a conjugate comprising the antibody or a cytotoxin in the preparation of an anticancer therapeutic agent.

Meanwhile, L-asparaginase (L-ASNase), an enzyme that catalyzes a reaction that hydrolyzes L-asparagine to produce aspartic acid and ammonia, was found to quickly and almost completely remove cancer cells in leukemia-induced mice, and was approved by the FDA in 1978 for the treatment of certain types of tumors that are primarily transmitted through the blood, such as acute lymphoblastic leukemia (ALL) and non-Hodgkin's lymphoma. Because T-lymphocytic leukemia cancer cells are unable to synthesize asparagine due to the reduced expression of asparagine synthase, they are required to rely on external sources of asparagine. Exposure to L-asparaginase (L-ASNase), which breaks down asparagine to aspartate, thereby kills T-lymphocytic leukemia cancer cells by inhibiting asparaginase supply and in turn protein synthesis. However, it has been reported that L-asparaginase, which works well against blood cancers, does not perform effectively against solid tumors. This is presumed to be due to the difficulty in delivering protein drugs to solid tumors, unlike in blood cancers. To enhance the tumor tissue delivery efficiency of L-asparaginase, the present inventors have previously developed an attenuated *Salmonella* strain genetically engineered to present L-asparaginase on its surface and reported its anticancer activity against solid tumors. (Korean Patent No. 10-1750007).

### DETAILED DESCRIPTION OF THE INVENTION

### TECHNICAL PROBLEM

However, the above prior art fails to tackle the problem of adverse health effects since it uses live bacteria, albeit attenuated. Therefore, there is a significant need to develop a new, safer asparaginase-based anticancer drug that has enhanced drug delivery efficiency to tumor tissue, which has been a drawback of existing recombinant proteins.

The present invention is intended to address the above and other problems, and aims to provide a novel asparaginase-based anticancer agent that target tumor antigens.

### TECHNICAL SOLUTION

In one aspect of the present invention, there is provided a fusion protein comprising a recombinant monobody which specifically binds to clareticulin and an L-asparaginase linked to the C-terminus of the recombinant monobody, wherein the recombinant monobody has a peptide that specifically binds to the calreticulin inserted into at least one of the BC loop and the FG loop of human fibronectin domain III (Fn3).

In another aspect of the present invention, there is provided a pharmaceutical composition for the treatment of solid tumors, comprising the fusion protein as an active ingredient.

In another aspect of the present invention, there is provided a method of treating solid tumors, comprising administering a therapeutically effective amount of the pharmaceutical composition to an individual with a solid tumor.

In another aspect of the present invention, there is provided a method of treating solid tumors, comprising administering a therapeutically effective amount of the fusion protein or the pharmaceutical composition to the individual with a solid tumor, simultaneously with or before or after irradiation.

### EFFECT OF THE INVENTION

The novel fusion protein of the present invention as described above is a very useful substance that can be used to effectively treat solid cancers that have not been easily treated by L-asparaginase. It can be used as a therapeutic agent for cancers, especially cancers characterized by the high expression of certain tumor antigens. However, these effects do not limit the scope of the present invention.

### BEST DESCRIPTION OF THE DRAWINGS

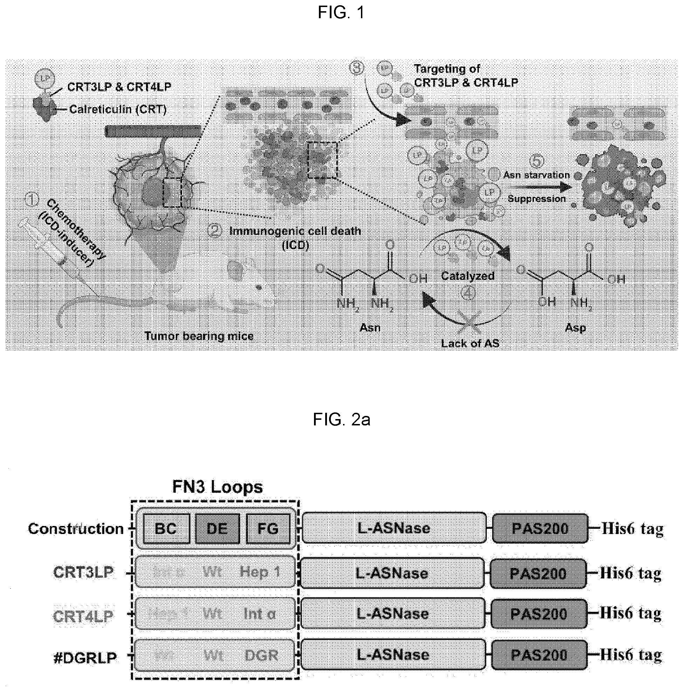

FIG. 1 is a schematic representation illustrating the mechanism of action of the PASylated CRT-targeted L-ASNases (CRT3LP and CRT4LP) of the present invention in mice treated with ICD-induced chemotherapy. ① Chemotherapy on tumor-bearing mice using ICD-inducing agents (anticancer agents). ② Exposure of ecto-CRT to ICD-induced tumor cells. (3) targeting tumor cells when treating with CRT3LP and CRT4LP and expsoing ecto-CRT. ④ Asn hydrolysis by L-ASNase activity in the tumor microenvironment, and blockage of Asn resynthesis due to AS dificiency in tumor cells. ⑤ Starvation and growth inhibition of tumor cells. CRT, calreticulin; ICD, immunogenic cell death; Asn, asparagine; Asp, aspartate; AS, Asn synthase.
FIG. 2a is a schematic diagram illustrating the structure of a PASylated CRT-targeted L-ASNase fusion proteins of the present invention. The L-ASNases were fused with monobodies and PAS200 tags at their N- and C-termini, respectively. The CRT3 and CRT4 monobodies contain CRT-targeted sequences [Int-α (KLGFFKR, SEQ ID NO: 15), Hep-I (GQPMYGQPMY, SEQ ID NO: 16)] in the BC and FG loops of the Fn3 backbone. The DE loop of each monobody is identical to that of wild-type Fn3 (Wt). The recombinant protein has a His6 tag sequence at the C-terminus and #DGRLP is a negative control containing a #DGR monobody, which is a Wt Fn3 protein with a DGR (scrambled monobody) instead of an RGD in the FG loop.
FIG. 2b is a schematic representation of the structure of CRT3LP as predicted by computer simulation. The CRT3 monobodies (blue) contain the CRT-targeted sequence in the BC and FG loops (left, top). After binding to the N-terminus, the four monobodies were positioned on the outside of the tetrameric L-ASNase complex (green) (left, bottom). The four PAS tags (pink) represent a random coiled conformation at the C-terminus of L-ASNase. The His6 tag was bound to the C-terminus of the PAS tag.
FIG. 2c is a gel photograph confirming the expression of PASylated L-ASNase in A. *coli.* Bacteria transformed with the expression plasmid were treated with IPTG inducer and proteins were separated by SDS-PAGE gel (left). Proteins transferred to the membrane were detected by Western blotting with anti-His tag antibody (right). Putative proteins expressed by IPTG induction were labeled (dashed box). Markers, protein size markers (kDa); -, without IPTG; +, with IPTG.
FIG. 2d is a graph representing the results of mass spectral analysis of the PASylated L-ASNase of the present invention. The purified protein was evaluated by mass spectrometry to determine its molecular weight (MW).

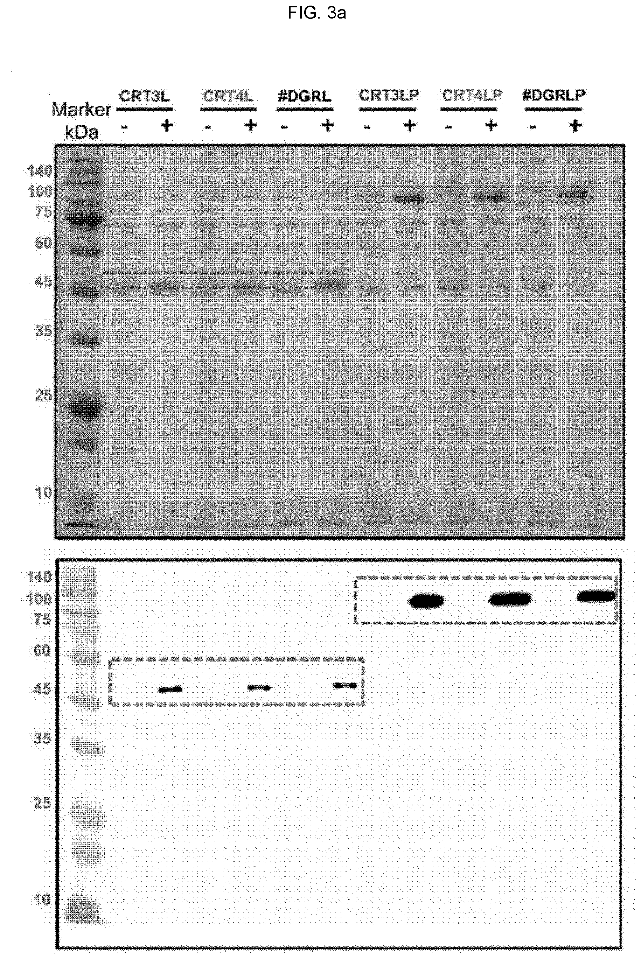

FIG. 3a is a gel photograph showing the results of determining the expression of monobody-bound L-ASNases with or without PASylation. *E. coli* were transformed with expression vectors carrying the monobody-bound L-ASNases with PASylation (CRT3LP, CRT4LP, and #DGRLP) or those without PASylation (CRT3L, CRT4L, and #DGRL), and induced with IPTG. +, with IPTG; -, without IPTG. Bacterial pellets (0.2 OD600 equivalent per lane) were separated by SDS-PAGE and stained with Coomassie blue (top panel). The proteins were detected by Western blot analysis using anti-His antibodies (lower panel). The putative proteins expressed by IPTG induction were marked (dotted boxes). Markers, protein size markers (kDa).
FIG. 3b is a graph showing the results of analyzing the relative expression levels of monobody-bound L-ASNases with and without PASylation. The density of protein bands was measured with Imaged software. Expression levels were calculated relative to #DGRL (fold).
FIG. 4a shows the purification and characterization of PASylated L-ASNases and is a gel photograph confirming the expression of purified PASylated L-ASNases by affinity chromatography. The proteins were separated by SDS-PAGE and stained with Coomassie blue (left). The separated proteins were validated by Western blotting with anti-His tag (right, top) and anti-L-ASNase antibodies (right, bottom), respectively. kDa, protein size marker. Throughout FIG. 4, #DGRLP (black), CRT3LP (blue), and CRT4LP (red) proteins are indicated by the corresponding colors, and L-ASNase (green) is the control.
FIG. 4b is a graph showing the results of the zeta potential analysis of PASylated L-ASNases.
FIG. 4c is a graph showing the results of analyzing the affinity of PASylated L-ASNases for CRT. The amount of CRT bound to PASylated L-ASNases was measured by ELISA at OD₄₅₀ and the affinity was described as the dissociation constant (Kd).
FIG. 4d is a graph representing the results of the enzymatic activity analysis of PASylated L-ASNases. Specific activity was expressed as IU per nmol of protein.
FIG. 4e is a graph showing the results of analyzing the relative thermal stability of PASylated L-ASNases. Enzyme activity at the indicated times was measured and calculated relative to 0 min.
FIG. 4f is a graph representing the results of an *in vivo* pharmacokinetic analysis of PASylated L-ASNases. Protein (10 IU) was injected intravenously into mice and the serum was collected. Residual protein was detected by ELISA at the indicated times. The amount of protein was converted to enzyme units. Free L-ASNase was used as a control. t1/2, half-life. *P < 0.05, ** *P* < 0.01, *** *P* < 0.001 and ns = not significant.

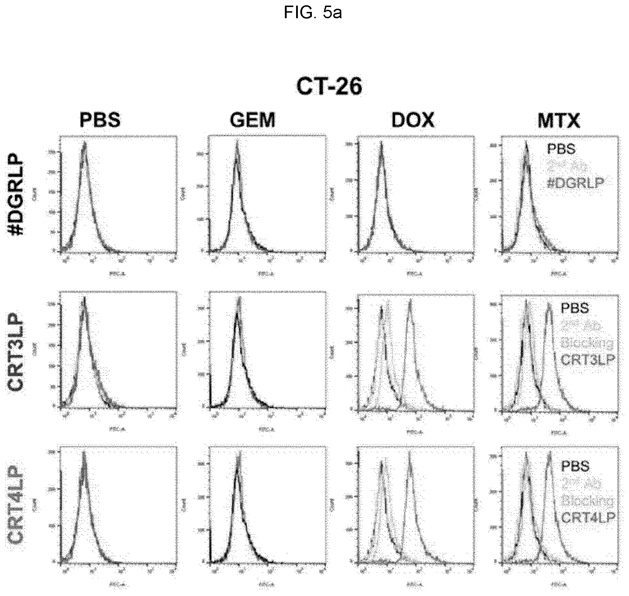

FIG. 5a is a flow cytometry histogram of CT-26 cells analyzed for binding of PASylated CRT-targeted L-ASNases to tumor cells treated with ICD-induced anticancer agents. CT-26 and MC38 cells treated with each anticancer drug (GEM, DOX or MTX) for 4h were stained with PASylated L-ASNases followed by anti-His antibodies and secondary antibodies conjugated with fluorescent dye. For blocking analysiss against ecto-CRT, the cells pretreated with anticancer agents were incubated with anti-CRT antibodies before staining with PASylated L-ASNases, PBS, and untreated cells. The cells incubated with the secondary antibodies conjugated with fluorescent dye alone were used as a control for background fluorescence.
FIG. 5b is a graph analyzing the results of a flow cytometry histogram of MC-38 cells.
FIG. 5c is a graph showing the results of analyzing the average fluorescence intensity of CT-26 cells.
FIG. 5d is a graph showing the results of analyzing the average fluorescence intensity of MC-38 cells.
FIG. 5e is an immunofluorescence image of PASylated L-ASNases in CT-26 cells treated with anticancer agents.
FIG. 5f is an immunofluorescence image of PASylated L-ASNases in MC-38 cells treated with anticancer agents. CT-26 and MC-38 cells were treated with anticancer agents for 4h. The cells were then stained with PASylated L-ASNases (100 nM) and anti-His tag antibodies, then examined by confocal laser scanning microscopy (40x magnification). Some areas of the images (boxes) were magnified 2x (80×). Green, ecto- CRT; blue, DAPI (nuclei); red, WGA (membrane). Scale bar, 50 µm. Data were presented as mean ± SD (n = 3). *** *P* < 0.001 and ns = not significant.
FIG. 6a is a graph showing the analysis of the cytotoxicity enhanced by CRT3LP and CRT4LP in tumor cells treated with ICD-induced anticancer agents and the viability of tumor cells treated with L-ASNases. CT-26 cells (4 × 10⁴) cultured overnight were treated with L-ASNases (200 IU/mL) in 96-well plates and viability was measured at the indicated times. The viability at each time was calculated relative to time 0. Cells were stained with anti-CRT antibodies.
FIG. 6b is a graph representing the results of flow cytometry histogram analysis for ecto-CRT. Filled black lines stained with isotype antibodies; red lines stained with anti-CRT antibodies.
FIG. 6c is a graph showing the results of viability analysis of CT-26 cells treated with CRT3LP and CRT4LP after pretreatment with anticancer agents.
FIG. 6d is a graph showing the results of viability analysis of MC-38 cells treated with CRT3LP and CRT4LP after pretreatment with anticancer agents. After washing, the cells were incubated with free or PASylated L-ASNases (1 IU/mL) for 24h. PBS was used as a control. Data were expressed as mean ± SD (n = 3). **P* < 0.05, *** *P* < 0.001 and NS = not significant.
FIG. 7a is a schematic illustration representing the experimental sequence for evaluation of the anti-cancer effect of CRT3LP in DOX-treated CT-26 tumor-bearing mice. The tumor-bearing mice (n = 3) were intraperitoneally injected with DOX three times (2 days apart). Simultaneously, the mice were injected intravenously with CRT3LP (0 - 20 IU per injection) five times (2 days apart). Two of the mice injected with 20 IU died within 12 days. PBS (untreated) and #DGRLP (8 IU per injection) were used as controls.
FIG. 7b is a graph showing the results of analyzing the tumor growth of individual mice in each group.
FIG. 7C is a graph showing the results of analyzing the tumor growth curves of individual mice in each group. Data were presented as mean ± SD.
FIG. 8a is a schematic illustration representing the experimental sequence for analyzing the enhanced anti-cancer efficacy of the combined use of ICD-induced chemotherapy and PASylated CRT-targeted L-ASNases in CT-26 tumor models. The CT-26 tumor-bearing mice were intraperitoneally injected with anticancer agents three times (2 days apart). In parallel, PASylated CRT-targeted L-ASNases (8 IU/injection) were injected intravenously (2 days apart). PBS and free L-ASNase were used as controls.
FIG. 8b is a representative image showing a tumor treated with both DOX and PASylated CRT-targeted L-ASNase.
FIG. 8C is a graph showing the results of analyzing the tumor growth in each experimental group.
FIG. 8d is a graph showing the results of analyzing the Kaplan-Meier survival curves for each group.
FIG. 8e is a photograph showing the size of tumors in each experimental group.
FIG. 8F is a graph showing the results of analyzing the average weight of tumors in each experimental group.
FIG. 8g is a representative immunohistochemical image of tumor tissue from each experimental group. Day 6 tumors were stained with PASylated L-ASNases and anti-His tag antibodies (green). DAPI (blue) was counterstained to see the nuclei. Images were taken at 40X magnification.
FIG. 8h is a graph representing the results of analyzing the signal intensity quantification of PASylated L-ASNases localized in tumor tissue. Semi-quantitative analysis of green fluorescent dots was performed by Image J software.
FIG. 8i is a graph showing the results of analyzing the alanine aminotransferase (ALT) levels in mouse serum from day 12. The yellow shaded area represents the average normal range (17 - 77 IU/L).
FIG. 8j is a graph showing the results of analyzing the aspartate aminotransferase (AST) levels in mouse serum from day 12. The yellow shaded area represents the average normal range (54 - 298 IU/L).
FIG. 8k is a graph showing the results of analyzing the anti-ASNase IgM levels in mouse serum from day 12. The amount of anti-ASNase IgM was measured by ELISA at OD₄₅₀.
FIG. 8l is a representative image of a CT-26 tumor treated with MTX and PASylated CRT-targeted L-ASNase.
FIG. 8m is a graph showing the results of analyzing the tumor growth of each experimental group.
FIG. 8n is a graph showing the results of analyzing the Kaplan-Meier survival curves for each experimental group.
FIG. 8o is a graph showing the results of analyzing body weight changes in CT-26 tumor-bearing mice after combined treatment with anticancer agents (DOX or MTX) and PASylated L-ASNases.
FIG. 8p is a graph representing the results of analyzing the tumor growth in CT-26 tumor-bearing mice after combined treatment with GEM and PASylated L-ASNases. Data were presented as mean ± SD (n ≥ 3).
FIG. 9 is a graph showing the growth curves of individual tumors in CT-26 mice treated with both DOX and PASylated L-ASNases (n = 5 - 8).
FIG. 10 is a graph showing the growth curves of individual tumors in CT-26 mice treated with MTX and PASylated L-ASNases (n = 5 - 9).
FIG. 11a is a representative image of a tumor in an MC-38 mouse treated with DOX and PASylated CRT-targeted L-ASNase, for analyzing the enhanced anti-cancer efficacy of the combined use of DOX and PASylated CRT-targeted L-ASNases in the MC-38 tumor models.
FIG. 11b is a graph showing the results of analyzing individual tumor growth curves for each group (n = 5 - 9).
FIG. 11c is a graph showing the average growth curves of the tumors in each group.
FIG. 11d is a graph showing the results of analyzing the Kaplan-Meier survival curves for each group.
FIG. 11e is a graph showing the results of analyzing the average weight change for each group. Data were presented as mean ± SD (n ≥ 5).
FIG. 12a is a graph depicting the results of cytotoxicity analysis as a function of time after irradiation of CT-26 cells. * *P* < 0.05, ** *P* < 0.01, *** *P* < 0.001, and ns = not significant.
FIG. 12b is a graph showing the results of analzying ecto-CRT over time after irradiation of CT-26 cells.
FIG. 12c is a graph showing the results of cytotoxicity analysis over time after irradiation of MC-38 cells.
FIG. 12d is a graph showing the results of analyzing ecto-CRT over time after irradiation of MC-38 cells.
FIG. 13a is a fluorescence image and graph showing the results of analyzing the binding of calreticulin-targeted L-ASNase after irradiation of CT-26 cells. * *P* < 0.05,** *P* < 0.01,*** *P <* 0.001, and ns = not significant.
FIG. 13b is a fluorescence image and graph showing the results of analyzing the binding of calreticulin-targeted L-ASNase after irradiation of MC-38 cells.
FIG. 14a is a representative microscopic image of CT-26 and MC-38 cells treated with calreticulin-targeted L-ASNases and stained with crystal violet after irradiation.
FIG. 14b is a graph representing the results of analyzing the cell viability after treating CT-26 cells with calreticulin-targeted L-ASNase after irradiation. * *P* < 0.05,** *P* < 0.01,*** *P <* 0.001, and ns = not significant.
FIG. 14c is a graph showing the results of cell viability after treating MC-38 cells with calreticulin-targeted L-ASNases after irradiation.

### BEST MODE FOR THE INVENTION

### Definitions of terms:

As used herein, the term "monobody" refers to a type of antibody mimetic, an artificial protein with a protein scaffold derived from the amino acid sequence of the tenth human fibronectin type III domain (FnIII). Monobodies are characterized by its ability to specifically bind to various antigens through amino acid modifications, so they are being used as a substitute for antibodies.

As used herein, the term "fibronectin" refers to a glycoprotein found in plasma and extracellular matrix (ECM) that has a structure comprising many of type I, type II, and type III domains with two anti-parallel beta-sheets structure linked to each other. The type I and type II domains are characterized by intrastructural disulfide bridges, which are absent in the type III.

The term "Calreticulin (CRT)" as used herein refers to a primary calcium storage protein in the sarcoplasmic reticulum of skeletal muscle. It binds calcium with low affinity but high capacity, binding approximately 25 calcium ions per molecule, and possesses a KDEL motif in its endoplasmic reticulum retention signal. It is also present in the nucleus of cells and is associated with DNA binding by nuclear hormone receptors and nuclear hormone receptor-mediated gene transfer. CRT binds to misfolded proteins and prevents them from escaping from the endoplasmic reticulum into the Golgi body. Recently, the importance of CRT in cancer has been gaining prominence. When cells are damaged by anticancer agents, the damaged cells expose CRTs on their cell surface to release "eat me signal", so that immune cells can eliminate the damaged cells. Since cancer cells have a feature of proliferating without stopping, as cancer progresses more CRTs appear on the surface of the cells, especially when treated with anticancer agents such as doxorubicin. The CRTs play a role in sending a pro-phagocytic signal to phagocytic macrophages, which indicates the message of "eliminate me (phagocytosis, cell lysis)" to immune cells in the blood.

As used herein, the term "tumor antigen" refers to an antigenic substance produced by cancer cells that is used as a target for the diagnosis and treatment of tumors. Tumor antigens are divided into tumor-specific antigens, which are found only on tumor cells, and tumor-associated antigens, which are also present in normal tissue but whose expression is significantly increased in tumor tissue.

As used herein, the term "peptide conjugate" refers to a formulation that allows for the prolonged efficacy of peptide pharmaceuticals, consisting of a complex formed by the combination of proteins and drugs

As used herein, the term "PASylation" refers to a biological substitution that has been utilized as an alternative to conventional PEGylation. It is a flexible, resinous sequence of 100 to 600 repeating proline (P), alanine (A), and serine (S) amino acids bound to the N- or C-terminus of a protein molecule. It significantly increases the hydrological volume of the macromolecule, thereby significantly extending its circulation time. It provides the benefits of PEGylation without affecting the biological activity or affinity of the target protein and facilitates biopharmaceutical manufacturing by eliminating the need for an *in vitro* binding step.

### A detailed description of the invention:

In one aspect of the present invention, there is provided a fusion protein comprising a recombinant monobody which specifically binds to clareticulin and an L-asparaginase linked to the C-terminus of the recombinant monobody, wherein the recombinant monobody has a peptide that specifically binds to the calreticulin inserted into at least one of the BC loop and the FG loop of human fibronectin domain III (Fn3).

In the fusion protein, the calreticulin is a tumor antigen, and another tumor antigens such as human ephrin type-A receptor 2 (EphA2), alpha-fetoprotein (AFP), carcinoembryonic antigen (CEA), CA-125, CA-19, CD19, CD20, CD22, Kappa-chain, CD30, CD123, CD33, LeY, CD138, CD5, BCMA, CD7, CD40, IL-1RAP, GD2, GPC3, FOLR (e.g., FOLR1 or FOLR2), HER2, EFGRVIII, IL13RA2, VEGFR2, ROR1, NKG2D, EpCAM, Mesothelin, MUC1, CLDN18.2, CD171, CD133, PSCA, cMET, PSA, EGFR, PSMA, FAP, CD70, MUC16, L1-CAM, B7H3, CAIX, adipophilin, AIM-2, ALDH1A1, alpha-actinin-4, ARTC1, B-RAF, BAGE1, BCLX(L), BCR-ABL fusion protein, beta-catenin, BING-4, CALCA, CASP-5, CASP-8, CD274, CD45, Cdc27, CDK12, CDK4, CDKN2A, CLPP, COA-1, CPSF, CSNK1A1, CTAG1, CTAG2, Cyclin D1, Cyclin-A1, dek-can fusion protein, DKK1, EFTUD2, elongation factor 2, ENAH (hMena), EpCAM, EphA3, epithelial tumor antigen (ETA), ETV6-AML1 fusion protein, EZH2, FGF5, FLT3-ITD, FN1, G250/MN/CAIX, GAGE-1,2,8, GAGE-3,4,5,6,7, GAS7, Glypican-3, GnTV, gp100/Pmel17, GPNMB, HAUS3, Hepsin, HER-2/neu, HERV-K-MEL, HLAA11, HLA-A2, HLA-DOB, hsp70-2, IDO1, IGF2B3, IL13Rα2, intestine carboxyl esterase, K-ras, Kallikrein 4, KIF20A, KK-LC-1, KKLC1, KM-HN-1, KMHN1, LAGE-1, LDLR-fucosyltransferase AS fusion protein, Lengsin, M-CSF, MAGE-A1, MAGE-A10, MAGE-A12, MAGE-A2, MAGE-A3, MAGE-A4, MAGE-A6, MAGE-A9, MAGE-C1, MAGE-C2, Malic enzyme, Mammaglobin-A, MART2, MATN, MC1R, MCSP, mdm-2, ME1, Melan-A/MART-1, Meloe, Midkine, MMP-2, MMP-7, MUC5AC, MUM-1, MUM-2, MUM-3, myosin, myosin class I, N-raw, NA88-A, neo-PAP, NFYC, NY-BR-1, NY-ESO-1/LAGE-2, OA1, OGT, OS-9, P polypeptide, p53, PAP, PAX5, PBF, pml-RAR alpha fusion protein, polymorphic epithelial mucin ("PEM"), PPP1R3B, PRAME, PRDX5, PTPRK, RAB38/NY-MEL-1, RAGE-1, RBAF600, RGS5, RhoC, RNF43, RU2AS, SAGE, secernin 1, SIRT2, SNRPD1, SOX10, Sp17, SPA17, SSX-2, SSX-4, STEAP1, survivin, SYT-SSX1 or -SSX2 fusion protein, TAG-1, TAG-2, telomerase, TGF-βRII, TPBG, TRAG-3, triosephosphate isomerase, TRP-1/gp75, TRP-2, TRP2-INT2, tyrosinease ("TYR"), VEGF, WT1, or XAGE-1b/GAGED2a besides the calreticulin may also be used.

In the fusion protein, the recombinant monobody may have a tumor antigen-specific binding peptide inserted into both the BC loop and FG loop, and may consist of an amino acid sequence represented by SEQ ID NO: 9 or 10. Furthermore, the recombinant monobody may have a calreticulin-binding peptide represented by SEQ ID NO: 15 inserted into the BC loop of the human fibronectin domain III and a calreticulin-binding peptide represented by SEQ ID NO: 16 inserted into the FG loop, or, it can have a calreticulin-binding peptide represented by SEQ ID NO: 16 inserted into the BC loop and a calreticulin-binding peptide represented by SEQ ID NO: 15 inserted into the FG loop.

In the fusion protein, the monobody may be screened using any one of the fibronectin domains as a scaffold, preferably derived from any one of the repeat domains of fibronectin type III. More preferably, the monobody may have a tumor antigen-specific binding peptide inserted into at least one of the BC loop and the FG loop of the tenth domain of fibronectin type III (FNIII10), or may have a tumor antigen-specific binding peptide inserted into both the BC loop and the FG loop of the FNIII10.

The fusion protein may further comprise a Pro-Ala-Ser (PAS) repeat, and the PAS repeat may be linked to an N-terminal or C-terminal end of the fusion protein. The PAS repeats may comprise from 20 to 500 units, such as 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, or a value between two or more units.

The fusion protein may comprise an amino acid sequence represented by SEQ ID NO: 23 or 24.

In another aspect of the present invention, there is provided a pharmaceutical composition for the treatment of solid tumors, comprising the fusion protein as an active ingredient.

The pharmaceutical composition may be used as an adjuvant to radiation therapy and may comprise one or more anticancer compounds, tumor suppressor proteins, or anticancer proteins. The anticancer compound may be a single substance or a combination of two or more selected from the group comprising immunogenic cell death agents, immune checkpoint inhibitors, mitotic inhibitors, antimetabolites, hormonal agents, alkylating agents, and topoisomerase inhibitors, wherein the immunogenic cell death agent may be anthracycline anticancer agents, taxanes, anti-EGFR antibodies, BK channel agonists, bortezomib, cardiac glycosides, cyclophosphamide agents, GADD34/PP1 inhibitors, LV-tSMAC, Measles virus, or oxaliplatin.

In the pharmaceutical composition, the anthracycline anticancer agent may be daunorubicin, doxorubicin, epirubicin, idarubicin, pixantrone, sabarubicin, or valrubicin. In addition to the anticancer compounds listed above, other anticancer compounds may be used alone or in combination with one or more of the compounds listed above, such as mecholrethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, DM1 (mertansine), DM4 (dolastatin), auristatin E, auristatin F, monomethyl auristatin E, monomethyl auristatin F, and derivatives thereof.

In the pharmaceutical composition, the tumor suppressor protein may be Von HippelLindau (VHL), Adenomatous polyposis coli (APC), cluster of differentiation 95 (CD95), Suppression of tumorigenicity 5 (ST5), Yippee like 3 (YPEL3), p53, Suppression of tumorigenicity 7 (ST7), or Suppression of tumorigenicity 14 (ST14).

In the pharmaceutical composition, the anticancer protein may be a protein toxin, an antibody specific for a cancer antigen or a fragment of the antibody, or an antiangiogenic factor. The protein toxin may be Botulinum toxin, Tetanus toxin, Shiga toxin, Diphtheria toxin (DT), ricin, Pseudomonas exotoxin (PE), cytolysin A (ClyA), or r-Gelonin. The tumor suppressor protein is a protein that inhibits tumor developments, such as von HippelLindau (VHL), Adenomatous polyposis coli (APC), cluster of differentiation 95 (CD95), Suppression of tumorigenicity 5 (ST5), Yippee like 3 (YPEL3), p53, Suppression of tumorigenicity 7 (ST7), and Suppression of tumorigenicity 14 (ST14). The antiangiogenic factors include, for example, anti-VEGF antibodies, angiostatin, endostatin, and the kringle V domain of apolipoproteins.

Furthermore, the anticancer protein may be a toxin, an antibody specific for a cancer antigen, or a fragment of the antibody, or an antiangiogenic factor, and the protein toxin may be Botulinum toxin, Tetanus toxin, Shiga toxin, Diphtheria toxin, DT), ricin, Pseudomonas exotoxin (PE), cytolysin A (ClyA), or r-Gelonin, and the antiangiogenic factor may be an anti-VEGF antibody, an angiostatin, an endostatin, a kringle V domain of an apolipoprotein, or the like.

In the pharmaceutical composition, the solid cancer may be lung cancer, stomach cancer, liver cancer, bone cancer, pancreatic cancer, gallbladder cancer, cholangiocarcinoma, skin cancer, head and neck cancer, skin melanoma, uterine cancer, ovarian cancer, rectal cancer, colon cancer, colorectal cancer, breast cancer, uterine sarcoma, fallopian tube carcinoma, endometrial carcinoma, cervical carcinoma, vaginal carcinoma, vulvar carcinoma, esophageal cancer, laryngeal cancer, small bowel cancer, or thyroid cancer.

In another aspect of the present invention, there is provided a method of treating an individual with a solid tumor, comprising administering a therapeutically effective amount of the pharmaceutical composition to the individual.

In another aspect of the present invention, there is provided a method of treating an individual with a solid tumor, comprising administering a therapeutically effective amount of the fusion protein or the pharmaceutical composition to the individual simultaneously with or before or after irradiation.

The pharmaceutical composition according to one embodiment of the invention may be administered to the individual by parenteral administration, wherein the parenteral administration may be by intravenous injection, intraperitoneal injection, intramuscular injection, subcutaneous injection, intracerebral injection, intraventricular injection, intracranial injection, intracerebrospinal injection, or intratumoral injection.

The pharmaceutical composition according to an embodiment of the present invention further comprises an inert ingredient, including a pharmaceutically acceptable carrier. As used herein, the term "pharmaceutically acceptable carrier" refers to a component of the composition, more specifically, a component other than the active substance of a pharmaceutical composition. Examples of pharmaceutically acceptable carriers include binders, disintegrating agents, diluents, fillers, glossing agents, solubilizing or emulsifying agents, and salts.

Furthermore, a pharmaceutical composition according to another embodiment of the present invention may be administered at a dosage of 0.1 mg/kg to 1 g/kg, more preferably at a dosage of 0.1 mg/kg to 500 mg/kg. However, the dosage may be appropriately adjusted according to the age, sex and condition of the patient.

In another aspect of the present invention, there is provided a method of treating an individual with a solid tumor, comprising administering a therapeutically effective amount of the fusion protein to the individual.

The present inventors prepared a recombinant CRT-monobody by substituting two CRT peptides, Int-α (SEQ ID NO: 15) and Hep-I (SEQ ID NO: 16), at each loop position (BC, DE, FG) of the conventional Fn3 domain, and confirmed that the anti-CRT monobody specifically binds to calreticulin. Then, to utilize the anti-CRT monobody as a therapeutic agent for calreticulin-overexpressing tumors, the present inventors prepared a fusion protein in which the anti-cancer protein L-ASNase was linked to the C-terminus of the anti-CRT monobody (FIG. 1). The present invention was completed by confirming that the fusion protein has a high killing capacity against cancer cells overexpressing calreticulin. In addition to the anti-CRT monobody, the present inventors also prepared a fusion protein with the structure of L-ASNase linked to an anti-EphA2 monobody that can specifically bind to another tumor antigen, EphA2. They confirmed that it also exhibited anticancer activity against tumors overexpressing EphA2, thus verifying that the tumor antigen-specific monobody-L-ASNase fusion protein of the present invention is a highly effective platform for anticancer therapy, especially for the treatment of solid tumors.

Specifically, bacterial L-ASNase has been widely used in the treatment of leukemia in combination with other anticancer agents (N. Verma, et al., Critical reviews in biotechnology, 27(1): 45-62, 2007). However, clinical trials designed to extend its application to solid tumors failed due to its limited accumulation in tumor tissue (F. Chiarini, et al., Biochimica et Biophysica Acta (BBA)-Molecular Cell Research, 1863(3): 449-463, 2016). Attempts to confer targeting activity to the enzymes were carried out by conjugation with target peptides. For example, L-ASNase conjugated with a target-specific scFv was designed (L. Guo, et al., Biochemical and Biophysical Research Communications, 276(1): 197-203, 2000). However, when the protein was expressed in *E. coli,* most of it was located in the inclusion body. A targeted-catalytic nanobody (T-CAN) has recently been reported (M. Maggi, et al, Cancers, 13(22): 5637, 2021), where the recombinant protein mentioned above contains a nanobody conjugated with a single subunit of L-ASNase that exhibits functional enzymatic activity, and binds to CD19-expressing ALL cells. However, the protein was also expressed as an inclusion body in *A. coli.* Because proteins such as scFv and nanobodies have an immunoglobulin-folded structure, the production of functional proteins from inclusion bodies in E. coli requires expensive processes such as separate refolding process. (A. Singh, et al, Microbial cell factories, 14(1): 1-10, 2015).

The present inventors showed that PASylated L-ASNases conjugated to CRT-targeting monobodies (CRT3LP and CRT4LP) are expressed in a soluble and functional form in *E. coli.* Indeed, these results were consistent with those obtained for Rluc8 conjugated to CRT monobodies. Combining monobodies and PAS200 tags with L-ASNases has several advantages. Since L-ASNases are tetrameric proteins, CRT3LP and CRT4LP have four monobody moieties at their N-terminus. Consistent with this, their affinity for CRT was four times higher than a single monobody. Furthermore, PASylation at the C-terminus increased their solubility; thus, these ligands were expressed much higher than non-PASylated ligands in *E*. *coli* and showed longer half-lives *in vivo.* This was consistent with the results of PASylated adnectin, which is a VEGFR2-targeting monobody. The increased *in vivo* half-life may be partly due to the protection of the enzyme by the N-terminal monobody moieties. L-ASNases inhibited mTORC1 signaling and induced cell cycle arrest in G1 phase, leading to apoptosis.

In the present invention, tumor cells treated with L-ASNases showed growth inhibition but did not detect ecto-CRT. Therefore, the cell death induced by the L-ASNase activity of CRT3LP and CRT4LP and the treated cells did not induce ecto-CRT. This indicates that CRT3LP and CRT4LP can only kill tumor cells exposed to external CRT. This was consistent with immunohistochemistry results, which showed in an irregular dotted pattern that CRT3LP and CRT4LP localized to DOX-treated tumors. High doses of CRT3LP (20 IU) caused sudden death in DOX-treated mice. The reason for this was first thought to be endotoxin contamination of the purified protein. For protein purification, a double chromatography procedure of His6 tag affinity and size exclusion was used, which could completely exclude endotoxin from the protein. There have been many protocols to exclude this contamination (S.J. Wakelin, et al., Immunology letters, 106(1), 1-7, 2006). The second possibility was an acute immune response triggered by CRT3LP. L-ASNase from bacteria can induce an immune response (A.M. Lopes, et al., Critical reviews in biotechnology, 37(1): 82-99, 2017). Monobody is derived from human Fn3. In addition, the PAS200 tag has been artificially synthesized and may also trigger an immune response (similar to PEG). The last possibility is ammonia generated by the L-ASNase activity of CRT3LP. Large amount of ammonia caused *in vivo* side effects such as cellular pH changes, mitochondrial membrane disruption, and ATP depletion. Although CRT3LP and CRT4LP have been tested against solid tumors, they could be clinically applicable to blood cancers such as ALL (acute lymphocytic leukemia). Currently, PEGylated L-ASNases have been used for blood cancer, although the number of PEG moieties is not clearly defined due to chemical bonding, and PEGylated L-ASNases do not have target specificity for blood tumors. When CRT3LP and CRT4LP are combined with chemotherapy for blood cancers may be more efficient than L-ASNase alone due to better CRT targeting.

The concept of "PASylated L-ASNase conjugated with monobody" can be extended to a variety of solid tumors. Many monobodies and other binding peptides bind specifically to tumor targets. Changes in monobody moieties may provide new target specificity for L-ASNases. In addition, clinical trials have been conducted in cancer patients using various enzymes that degrade specific amino acids (H. Komuro, et al., Bioengineering, 9(2): 56, 2022). Other therapeutic proteins (e.g., toxins, cytokines, and immunogens) can be used in place of L-ASNases for drug development.

The present inventors observed the efficacy of PASylated CRT-targeted L-ASNase as an anticancer agent in solid tumors treated with ICD-guided chemotherapy. L-ASNase, an enzyme used as an anticancer agent against hematologic cancers, can be applied to solid tumors through conjugation with target-specific monobodies. More specifically, L-asparaginase (L-ASNase), a bacterial enzyme that breaks down asparagine, has been commonly used in combination with several chemical drugs to treat malignant hematopoietic cancers such as acute lymphocytic leukemia (ALL). In contrast, while the above enzymes are known to inhibit the growth of solid tumor cells *in vitro,* they are not effective *in vivo.* In the prior invention, the present inventors reported that two novel monobodies (CRT3 and CRT4) specifically bind to calreticulin (CRT) exposed on tumor cells and tissues during immunogenic cell death (ICD). Subsequently, the present inventors designed L-ASNase conjugated to the monobodies at the C-terminus (CRT3LP and CRT4LP), N-terminus and the PAS200 tag. The proteins were expected to have four monobodies and PAS200 tag moieties that would not interfere with the L-ASNase conformation. The proteins were expressed 3.8-fold higher in *E*. *coli* than those without PASylation. The purified proteins were highly soluble with a much larger apparent molecular weight than expected. The affinity (Kd) of the proteins for CRT was approximately 2 nM, which is 4 times higher than that of a monobody. The enzymatic activity (~6.5 IU/nmol) was similar to that of L-ASNase (~7.2 IU/nmol) and the thermal stability increased significantly at 55°C. The half-life was > 9h in mouse serum, roughly 5-fold longer than that of L-ASNase (1.8h). In addition, CRT3LP and CRT4LP specifically bound to CRTs exposed to tumor cells *in vitro,* significantly inhibiting tumor growth in CT-26 and MC-38 tumor-bearing mice treated with ICD-inducing drugs (doxorubicin and mitozantrone), but not with non-ICD-inducing drugs (gemcitabine). All data indicate that PASylated CRT-targeted L-ASNases enhanced the anti-cancer efficacy of ICD-guided chemotherapy. Therefore, the L-ASNase showed a potential to be an anticancer agent for treating solid tumors, and the anticancer effect of PASylated calreticulin-targeted L-ASNases in mice bearing solid tumors by immunogenic apoptosis-inducing chemotherapy was demonstrated (FIG. 1).

The present invention will now be described in more detail with reference to the following examples. However, the present invention is not limited to the embodiments disclosed herein, but may be embodied in many different forms, and the following embodiments are provided to make the disclosure of the invention complete and to give those of ordinary skill in the art a complete idea of the scope of the invention.

### MATERIALS AND METHODS

### Cell lines and reagents

The mouse colon cancer CT-26 and MC-38 cell lines used in the present invention were purchased from American Type Culture Collection (ATCC, USA) and Kerafast, USA, respectively. DMEM medium was purchased from GIBCO, USA, and, fetal bovine serum (FBS) and phosphate buffered saline (PBS) were purchased from Gibco/Thermo Fisher Scientific, USA. Also, penicillin/streptomycin solution was purchased from Sigma-Aldrich, USA. Asparaginase activity analysis kit and recombinant calreticulin protein (rCRT) were purchased from Abcam, USA, and paraformaldehyde (PFA) was purchased from Biosesang, Korea. L-ASNase was purchased from Prospec, USA, and Cell Counting Kit-8 (CCK-8) was purchased from Enzo Life Sciences, USA. All antibodies used in the present invention are summarized in Table 1 below.

**Table 1**

| Information of used antibodies | | |
|---|---|---|
| Antibody name | Company/Catalog Number | Remarks |
| Calreticulin (D3E6) XP Rabbit mAb | Cell signaling/ #12238 | 1:400 dilution |
| L-asparaginase polyclonal antibody | Thermo scientific/ 200-4171-0100 | 1:500 - 5000 dilution |
| Recombinant anti-6X His tag antibody | Abcam/ab245114 | 1:500 - 2000 dilution |
| Anti-rabbit secondary | Invitrogen/3 1460 | 1:2000 dilution |
| antibody, HRP | | |
| Asparaginase antibody (HRP) | GeneTex/GTX40848 | 1:700 dilution |
| Goat anti-rabbit secondary antibody, Alexa Flour 488 | Thermo scientific/ A-11008 | 5 µg/mL |
| Wheat germ agglutinin - Alexa Flour 555 Conjugate | Thermo scientific/ W32464 | 1:5000 dilution |
| Goat anti-mouse IgM (Heavy chain) secondary antibody, HRP | Thermo scientific/ 62-6820 | 1:2000 dilution |

### Construction of an Expression Vector Delivering PASylated L-ASNase

The PAS200 fragment (ASP AAP AP ASP AAP AP APSAP A) 1 0 was referenced to the prior art (J. Breibeck, et al., Biopolymers, 109(1): e23069, 2018) and based on the use of *E. coli* codons, the amino acid sequence was back-translated by the EMBOSS Backtranseq program (EMBL's European Bioinformatics Institute, UK) to yield the gene. Sequences corresponding to the *Eco*R1 and *Bam*H1 sites were added to the 5' and 3' ends, respectively, and some nucleotides were changed to create the *Pst*1 site without mutation of amino acids in the middle of the PSA200 gene. Based on the above gene sequences, two fragments, *Eco*R1-PAS100-*Pst*1 and *Pst*1-PAS100-*Bam*H1, were chemically synthesized (Macrogen, Korea). The fragments were digested with the corresponding restriction enzymes and triple ligated with plasmid pETh-E1-EGFP digested with *Eco*R1 and *Bam*H1 (M.A. Kim, et al., PloS one, 12(7): e0180786, 2017). The resulting plasmid was named pETh-E1-PAS200. The full-length PAS200 fragment was used as a template for the polymerase chain reaction (PCR) with the primers BglII-G4S-PAS200-F (SEQ ID NO: 30) and pET-R (SEQ ID NO: 31) for pETh-E1-PAS200. After phosphorylation with T4 polynucleotide kinase, the fragments were cloned into pBluescript II SK(+) (Agilent Technologies, USA), digested with EcoRV and dephosphorylated with alkaline phosphatase (Thermo Fisher Scientific, USA). The resulting plasmid was named pBS-BglII-G4S-PAS200-BamH1. The plasmid pASN containing *E. coli* L-ASNase was obtained from Dr. Hyun-Yi Choi (Chonnam National University College of Medicine) (K. Kim, et al., Molecular Therapy-Oncolytics, 2, 15007, 2015). The above L-ASNase gene fragment was PCR amplified using LASP-EcoR1-F (SEQ ID NO: 32) and LASP-*Bam*H1*-R* (SEQ ID NO: 33) primers for pASN as template. The above fragments were cut *into Eco*R1 and *Bam*H1*,* cloned into the EcoR1-BglII site of pBS-BglII-G4S-PAS200-*Bam*H1, and the resulting plasmid was named pBS-LASP-PAS200. Expression vectors for CRT-targeting monobodies (CRT3 and CRT4) and their isotype controls (#DGR) [pETh-CRT3, pETh-CRT4, and pETh-Fn3(DGR)] were referenced to the prior art (Y. Zhang, et al., Cancers, 13(11): 2801, 2021). The above monobody genes were PCR amplified by T7 (SEQ ID NO: 34) and 94old (GC)-R (SEQ ID NO: 35) primers with the respective corresponding plasmids as template, and then cut *with Nhe*1 and *Eco*R1. LASP-PAS200 fragments were prepared by digestion of pBS-LASP-PAS200 with *Eco*R1 and *Bam*H1*.* The respective monobodies and LASP-PAS200 gene fragments were triple-ligated into pETh-E1-PAS200 digested with *Nhe1* and *Bam*H1*,* and the resulting plasmids were named pETh-CRT3-LASP-PAS200, pETh-CRT4-LASP-PAS200, and pETh-Fn3(DGR)-LASP-PAS200. They have genes encoding PASylated L-ASNase proteins named CRT3LP, CRT4LP, and #DGRLP, respectively. As a result, the genes for the monobodies, L-ASNases, and PAS200 tags were ligated via a (G₄S)₂ linker and contained a His6 tag at the C-terminus. All plasmids described above were confirmed by sequencing (Macrogen, Korea). The nucleic acid sequences of the primers used for the above cloning are summarized in Table 2.

**Table 2**

| Information of nucleotide sequences | | |
|---|---|---|
| Primer | Sequence 5'-> 3' | SEQ ID NOs: |
| BglII-G4S-PAS200-F | acaagatctagcgccgccgccgccgccagcgccgccgccgc cgccagcgctagtccagccgctccagcacctgc | 30 |
| pET-R | tttttgctcagcggtggcagcagcc | 31 |
| LASP-EcoR1-F | ggcagcgaattcttacccaatatcaccattttag | 32 |
| LASP-B amH 1-R | gccgctggatccgtactgattgaagatctgctggat | 33 |
| T7 | taatacgactcactatagggggaattg | 34 |
| 94old(GC)-R | caggccctgcagaagcttgaattcgctgccgccgccgccgctg ccgccgccgccgccgcttgttcggtaattaatggaaatttggg | 35 |

### Purification and characterization of recombinant proteins

The recombinant proteins were purified by reference to the prior art (Y. Zhang, et al., Cancers, 13(11), 2801, 2021). In summary, *E. coli* BL21(DE3) (Invitrogen, CA) transformed with expression vectors was cultured overnight at 37°C in LB medium containing 50 µg/mLkanamycin. Bacteria were inoculated into 500 mL of fresh LB broth containing 50 µg/mLkanamycin (diluted to 1/100). After 3h of incubation at 37°C, the bacteria were treated with isopropyl-β-D-thiogalactopyranoside (IPTG) at a final concentration of 0.5 mM. After incubation for another 4h, the bacterial pellets were harvested by centrifugation at 8,000 xg for 5 minutes at 4°C and resuspended for 30 minutes in ice-cold lysis buffer [50 mM NaH₂PO₄, 300 mM NaCl, and 10 mM imidazole (pH 8.0)] containing 100 µg/mLof lysozyme solution, and then gently sonicated on ice. After centrifugation, the supernatant was purified using a His GraviTrap column (GE Healthcare, USA) and excess imidazole was removed with a PD-10 desalting column (GE Healthcare, USA). The proteins were then dispersed in PBS and stored at 4°C until needed.

### Computer modeling of the CRT3LP structure

The structure of a CRT3 monobody was constructed using AlphaFold (J. Jumper, et al., Nature, 596(7873), 583-589, 2021). The highest ranked structure was selected for visualization. The structure of CRT3LP was built by superimposing the four units of a CRT3 monobody on the crystal structure of a L-ASNase tetramer (PDB 2HIM). All protein structures were rendered and analyzed using PyMOL (W.L. DeLano, et al., Protein Crystallogr, 40(1): 82-92, 2002).

### SDS-PAGE and Western blot analysis

The bacterial pellets of purified proteins were mixed with 5x sample buffer (ElpisBio, Korea) and loaded onto 12% SDS-PAGE gels (10 µg/well). After separation by electrophoresis, the proteins were visualized with Coomassie blue R-250 stain (Enzynomics, Korea). Western blotting was performed to evaluate protein expression in bacteria and the purity of the recombinant proteins. In summary, proteins from SDS-PAGE gels were transferred to nitrocellulose membranes at 4°C for 100 min at 120 volts. The membrane was then incubated in Tris-buffered saline-0.1% Tween^{®}20 (TBS-T) solution containing 5% skim milk for 1h at room temperature. The washed membrane was incubated with horseradish peroxidase (HRP)-conjugated anti-rabbit secondary antibodies (diluted to 1:2000) for 1h. The immunoblotted proteins were detected using a LAS-3000 chemiluminescence detection system (Fuji, Japan).

### Mass Spectrometry

The molecular weights of the recombinant proteins were determined by Autoflex speed MALDI-TOF/TOF mass spectrometry at Gwangju Institute of Science and Technology (GIST) Central Research Facility (GCRF, Korea).

### Zeta Potential Measurement

The zeta potential values of the recombinant proteins were measured with a zeta potential and particle size analyzer ELS-Z series (Otsuka Electronics, Japan). Measurements were performed in triplicate on a purified sample [50 µg of protein in 800 µL of distilled water (pH 7.2)] at 25 °C.

### Binding affinity

The affinity of the recombinant protein for CRT was measured by enzyme-linked immunosorbent analysis (ELISA) (J.D. Beatty, et al., Journal of immunological methods 100(1-2), 173-179, 1987). In brief, 100 µL of PASylated CRT-targeted L-ASNases coated onto 96-well microplates (0 - 10 µM/well) overnight at 4 °C. Unbound proteins were removed by aspiration from the wells and 100 µL of 10 µM rCRT proteins were added to each well and incubated for 2h at room temperature. After rCRTs were removed, rabbit anti-CRT antibodies (diluted to 1:500) were added to each well and incubated for 2h at room temperature. The antibodies were removed by aspiration, and the wells were washed five times with T-PBS. Then, biotin-conjugated anti-rabbit secondary antibodies (diluted to 1:1000) were added and incubated for 1h. After washing with T-PBS, 100 µL of avidin-HRP (diluted to 1:250) was added to each well and incubated for 30 minutes at room temperature. After aspiration and washing five times, 100 µL of 1x3,3',5,5'-tetramethylbenzidine (TMB) buffer (Thermo Fisher Scientific, USA) was added to each well to form a yellow reaction product. After 15 minutes, 50 µL of 0.5 M H2SO4 solution was added to each well to stop the chromogenic reaction. The amount of colorimetric product was evaluated as the optical density value at 450 nm (OD450) by a SpectraMax M2 microtiter plate reader (Molecular Devices, USA).

### L-ASNase Activity Analysis

The L-ASNase enzyme activity of the recombinant proteins was measured with an asparaginase activity analysis kit using an OxiRed probe (Abcam, USA). 1 International unit (IU) was defined as the amount of enzyme generating 1.0 µmol of Asp per minute at 25 °C.

### Thermal stability analysis

Briefly, 200 µL of PASylated recombinant protein or L-ASNase (1 mg/mL) was incubated in a thermal cycler (Finepcr, Korea) at 55°C for 30-180 min. The residual enzymatic activity of the proteins was measured using an asparaginase activity analysis kit as described above. The residual activity (%) at each incubation time was calculated relative to the sample activity at time 0.

### Flow cytometry

Cells were cultured at 37°C in DMEM medium supplemented with 10% FBS and 100 U of streptomycin/penicillin in an atmosphere of 5% CO2. Cells were treated with each anticancer drug [3 µM methotrexate (MTX), 25 µM doxycycline (DOX), or 15 µM gemcitabine (GEM)] to induce apoptosis (Y. Zhang, et al., Cancers, 13(11), 2801, 2021). After 4h of incubation, the cells were scraped and detached from the plate. The cells (10⁵) were fixed with 1% PFA for 15 min and then treated with 100 nM of PASylated recombinant proteins for 1h on ice. After washing five times with cold PBS buffer containing 0.1% Tween-20 (T-PBS), the cells were incubated with anti-His tagged monobodies (diluted to 1:1000) for 1h and then treated with Alexa Fluor 488-conjugated secondary antibodies (5 µg/mL) for 1h. Fluorescence signals were detected by FACSCanto II Flow Cytometer (BD Biosciences, USA). For blocking experiments, cells were first incubated with anticancer agents for 4h and fixed with 1% PFA. Cells were then incubated with anti-CRT antibodies (diluted to1:400) for 1 h to mask ecto-CRT. Cells were stained with PASylated recombinant proteins and followed the same procedure as described above.

### Confocal immunofluorescence imaging analysis

PASylated CRT-targeted L-ASNases bound to ecto-CRT on cells were visualized by confocal immunofluorescence microscopy with reference to the prior art (Y. Zhang, et al., Cancers, 13(11), 2801, 2021). After overnight incubation in a cover-glass, CT-26 and MC-38 cells (104) were treated with anticancer agents for 4 h at 37 °C. The cells were then fixed with 1% PFA for 15 min, stained with 100 nM PASylated recombinant proteins for 1h on ice, and treated with anti-His tagged monobodies (diluted to 1:1000) and Alexa Fluor 488-conjugated secondary antibody for 1h (5 µg/mL). Cell membranes were stained with Alexa Fluor 555-conjugated wheat germ agglutinin (WGA) (diluted to 1:5000). Between each step, samples were washed with cold T-PBS. Finally, the cells were incubated with 4',6-diamidino-2-phenylindole (DAPI) (Thermo Fisher Scientific, USA) to stain the nuclei. Immunofluorescence signals were imaged using an LSM510 confocal microscope (ZEISS, Jena, Germany) and data were analyzed with ZEN-LSM imaging software (ZEISS, Jena, Germany).

### Cell viability

CT-26 and MC-38 cells (104 cells per well) were seeded into three wells of a 96-well plate and incubated at 37°C. The next day, the culture medium was replaced with 100 µL of fresh medium containing the above concentrations of anticancer agents. After incubation at 37°C for 4 h, the culture medium was removed and the cells were washed three times with PBS. The cells were then incubated with 100 µL of L-ASNase or PASylated recombinant protein (1 IU/mL in DMEM medium) for 24h. Finally, CCK-8 reagent (10 µL/well) was added for 3h at 37 °C, and the amount of color in each sample was measured at OD450.

### In vivo pharmacokinetic analysis

Six-week-old female BALB/c mice (Orient Company, Korea) were randomly assigned to different treatment groups (n = 3) and intravenously injected with 10 IU of PASylated recombinant protein. Then, blood samples were taken from the eyes at the specified time (0 - 52h) and left at 4°C for 40 min. After centrifugation at 12,000 rpm for 20 min, the serum fraction was obtained and stored at -80°C before further analysis. PASylated recombinant protein in serum was assessed using the ELISA method described above. The amount of serum recombinant protein was converted to enzyme activity.

### In vivo anti-cancer efficacy analysis

CT-26 and MC-38 tumor cells (10⁶ in 100 µL PBS) were implanted subcutaneously into the right flanks of 6-week-old BALB/c and C57BL/6 female mice (Orient company, Korea), respectively. When the tumors reached about 100 mm³, the tumor-bearing mice were injected intraperitoneally with anticancer agents (15 mg/kg GEM, 10 mg/kg DOX or 2 mg/kg MTX) three times every 2 days. Simultaneously, the mice were injected intravenously with the PASylated recombinant proteins of the present invention five times every 2 days. The length (L), width (W), and height (H) of each tumor were recorded every 3 days using a digital caliper, and the tumor volume (mm3) was calculated using the following formula (L x H x W)/2. Mice were euthanized when tumors were ~1,500 mm³ and all animal experiments were performed in accordance with the general principles and procedures outlined in the National Institutes of Health Guidelines, and all protocols were approved by the Chonnam National University Animal Care and Use Committee (permit number: HCRL 16-001).

### Statistical analysis

All data are expressed as mean ± standard deviation (SD), and statistical analysis was performed using GraphPad Prism 5.0 (GraphPad, USA). Survival analysis was performed using the Kaplan-Meier method and log-rank test. *P*-values <0.05 were considered significant (*), <0.01 was considered highly significant (**), and <0.001 was considered highly significant (***).

### Example 1: Design and characterization of PASylated CRT-targeted L-ASNases

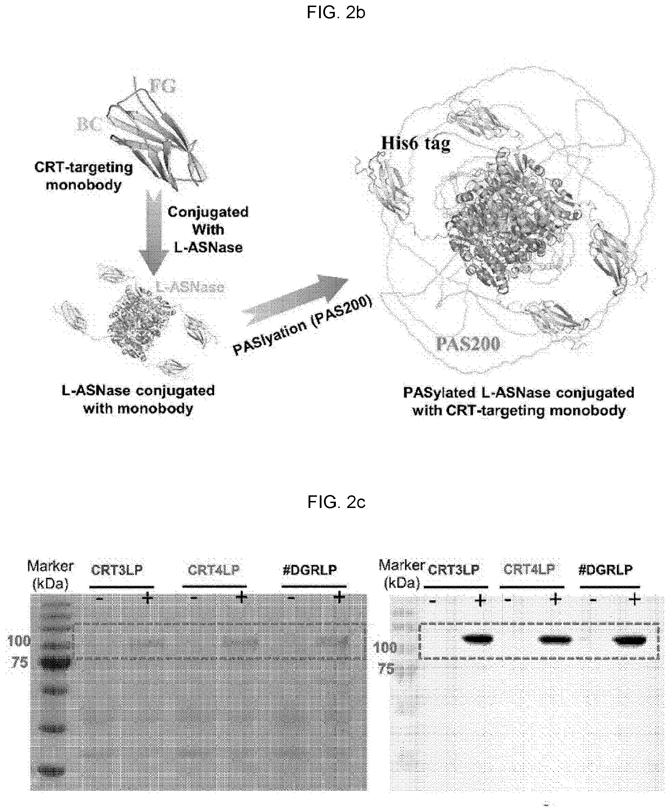

The CRT-targeting monobodies (CRT3 and CRT4) and control (#DGR) were designed to be fused with L-ASNases and PAS200 tags (FIG. 2a). The recombinant proteins were named CRT3LP, CRT4LP, and #DGRLP, respectively. All proteins were expected to have a His6 tag at the C-terminus. The structure of CRT3LP was estimated by computer simulation (FIG. 2b). Because L-ASNase is a homotetramer and the N-terminus of each enzyme subunit is exposed outside the enzyme complex, four CRT3 monobodies were linked to the enzyme complex. The PAS200 tag at the C-terminus of each enzyme subunit was also exposed to the outside and surrounded the enzyme complex. Based on the simulated structure, the CRT3 monobody was expected to maintain binding specificity and not affect the L-ASNase structure of the recombinant protein. The PAS200 tag was expected to protect the enzyme complex from protease attack. The His6 tag was also exposed, suggesting that purification by affinity chromatography would be efficient.

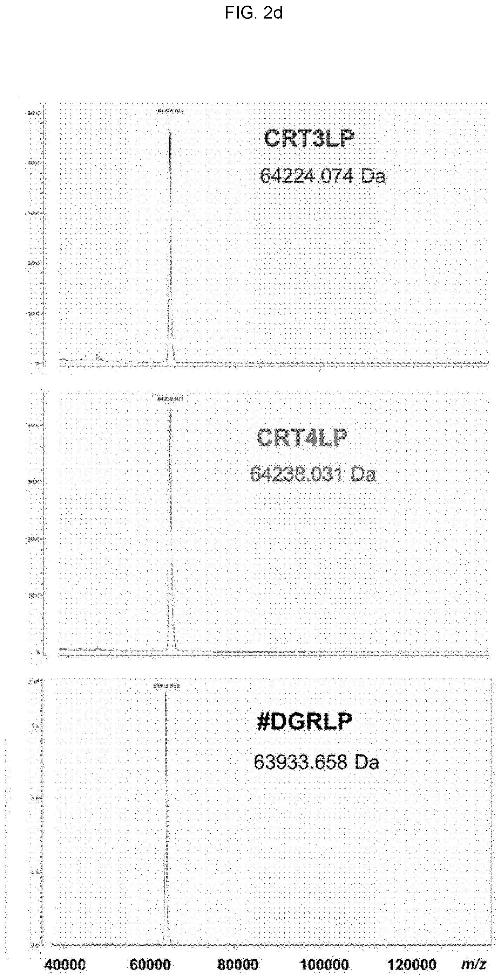

The recombinant proteins were expressed in *E. coli* transformed with the expression vector after IPTG induction (FIG. 2c). The recombinant proteins were detected only in the presence of IPTG. However, although the expected molecular weight (MW) was approximately 64 kDa, the MW of the bands detected in the SDS-PAGE gel and Western blot was estimated to be more than 100 kDa. To explain the discrepancy, the recombinant proteins were purified and the actual MW was measured by mass spectrometry (FIG. 2d), and the measured value was the same as the expected MW.

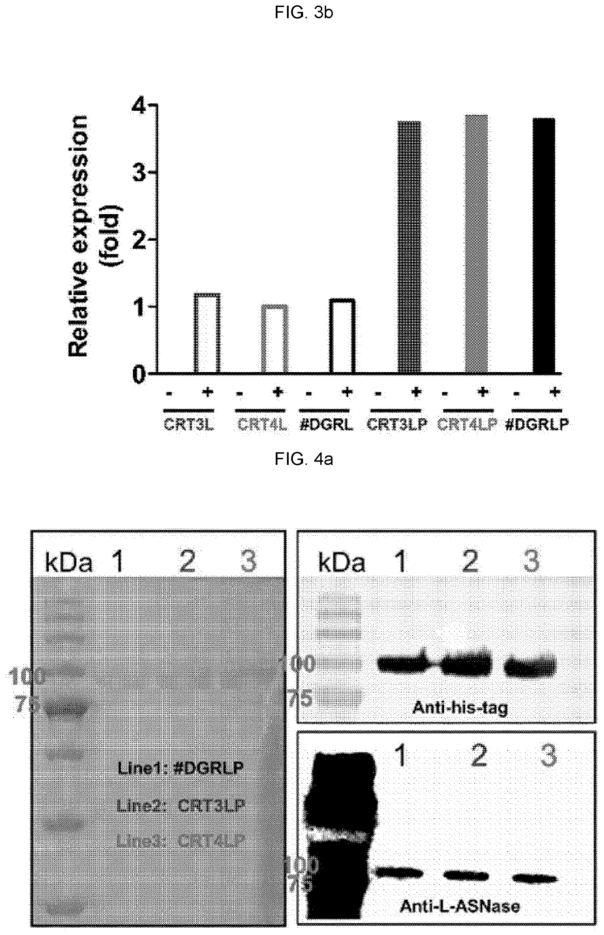

These results are similar to prior reports that PASylation increases protein hydrodynamic volume through modification of surface hydrophilicity, resulting in decreased electrophoretic mobility in gel electrophoresis (S. Aghaabdollahian et al., Scientific reports, 9(1): 1-14, 2019). Furthermore, the PASylated proteins were expressed 3.8-fold higher than non-PASylated proteins in A. *coli* (FIGS. 3a and 3b). Since high expression of recombinant proteins in bacteria often leads to the formation of inclusion bodies (A. Singh, et al., Frontiers in Microbiology, 11, 876, 2020), the above results indicate that PASylation increased protein solubility to reduce aggregation and ultimately increased production yields.

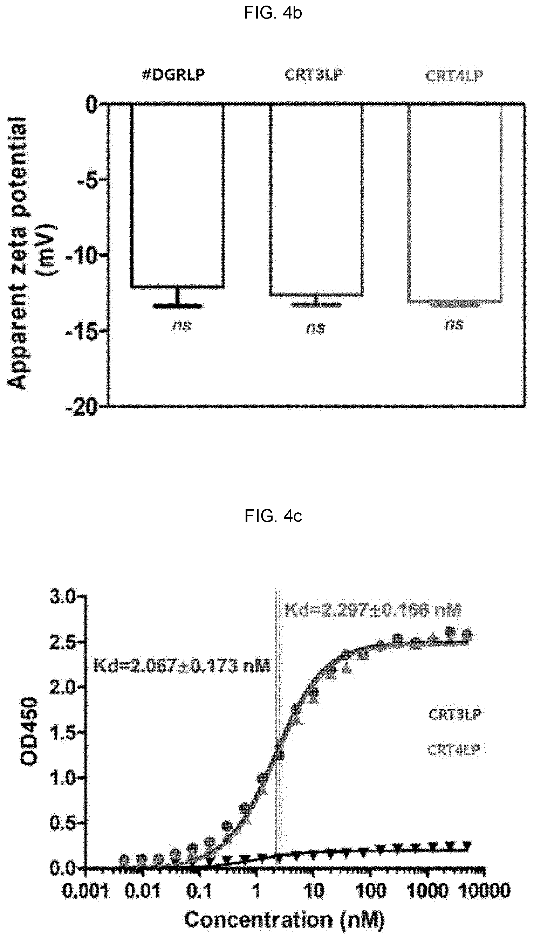

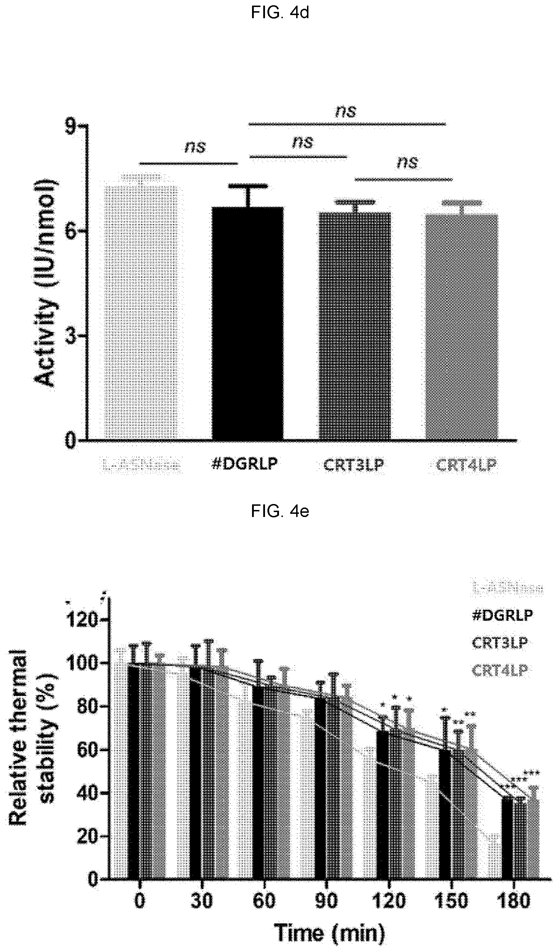

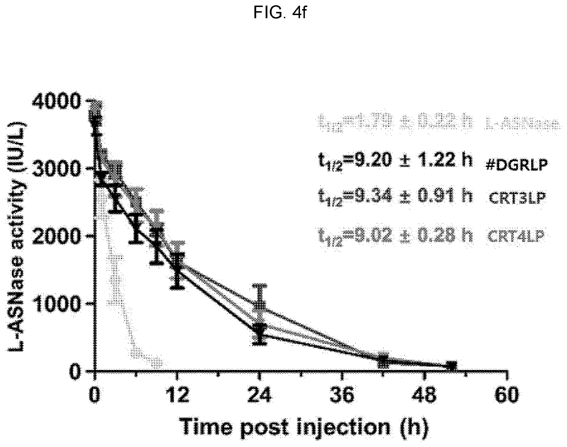

Next, the PASylated CRT-targeted L-ASNases were characterized (FIG. 4). In SDS-PAGE, the purified proteins showed a migration pattern similar to that of the bacterial pellets shown in FIG. 2c with a MW exceeding 100 kDa (FIG. 4a, left panel). The proteins were detected by anti-His tag and anti-L-ASNase antibodies in Western blot analysis (FIG. 4a, upper and lower panels). The zeta potential of the recombinant proteins was measured in the aqueous phase (FIG. 4b). Since the recombinant proteins have an isoelectric point (pIs) of 5.45, it was expected to be negatively charged in aqueous solution. The zeta potential (approximately -12.5 mV) was consistent with this expectation.

Furthermore, the affinity of the recombinant proteins for CRT was evaluated by ELISA analysis (FIG. 4c). The dissociation constant (Kd) values were 2.067 ± 0.173 nM for CRT3LP and 2.297 ± 0.166 nM for CRT4LP, respectively. The above values were similar to those of typical monobodies. The affinity of the proteins was four times higher than that of CRT3 and CRT4 monobodies (Y. Zhang, et al., Cancers, 13(11): 2801, 2021). As expected, #DGRLP did not show binding affinity for CRT. The enzymatic activity of the PASylated CRT-targeting L-ASNase was measured (FIG. 4d). The enzymatic activity was similar to that of L-ASNase (~6.5 IU/nmol for PASylated protein vs. ~7.2 IU/nmol for L-ASNase). These results indicate that the monobody and PAS200 tags have little interference with the enzymatic activity of PASylated L-ASNase. Since the thermal stability of the enzyme is an important parameter for in vivo application, the enzyme activity was measured at 55°C for 180 min (FIG. 4e). The relative activity of the PASylated protein gradually decreased over time; however, the decrease was less than that observed for L-ASNase. After 180 min, the residual enzymatic activity of the PASylated protein was approximately 35.1% of the initial activity, whereas the residual enzymatic activity of free L-ASNase was 16.4%. This is consistent with the results obtained with the monobody and PAS200 tags (A. Skerra, et al., Journal of Molecular Recognition, 13(4): 167-187, 2000), likely due to the restriction of the L-ASNase construct given by the fusion of monobody with the PAS200 tag. Finally, we evaluated the pharmacokinetics of the PASylated proteins in BALB/c mice (FIG. 4f). The PASylated proteins or L-ASNases (10 IU) were injected via tail vein, and serum samples were collected at the indicated times. The PASylated recombinant proteins showed a ~5-fold improvement in half-life: #DGRLP, CRT3LP, and CRT4LP had half-lives of 9.20 ± 1.22h, 9.34 ± 0.91h, and 9.0 2± 0.28h, respectively, compared to 1.79 ± 0.22h for L-ASNase.

### Example 2: Cytotoxicity against tumor cells

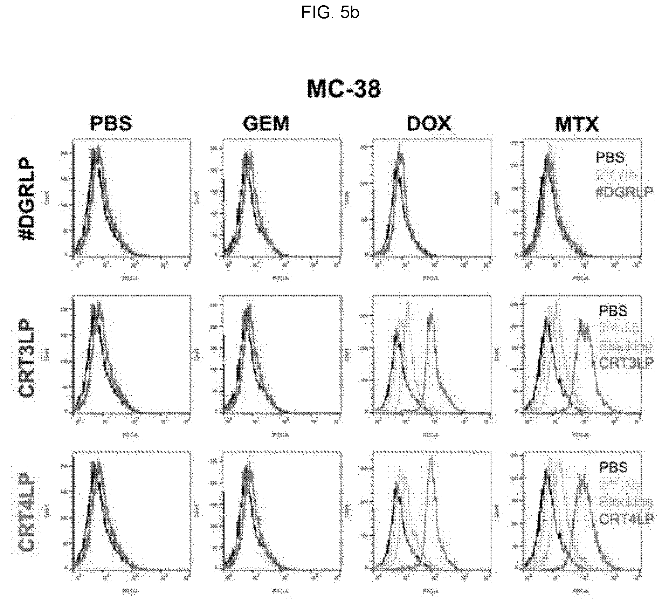

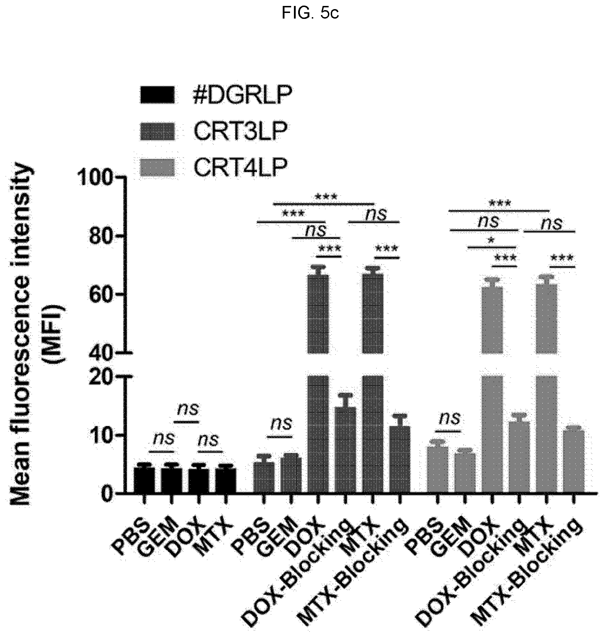

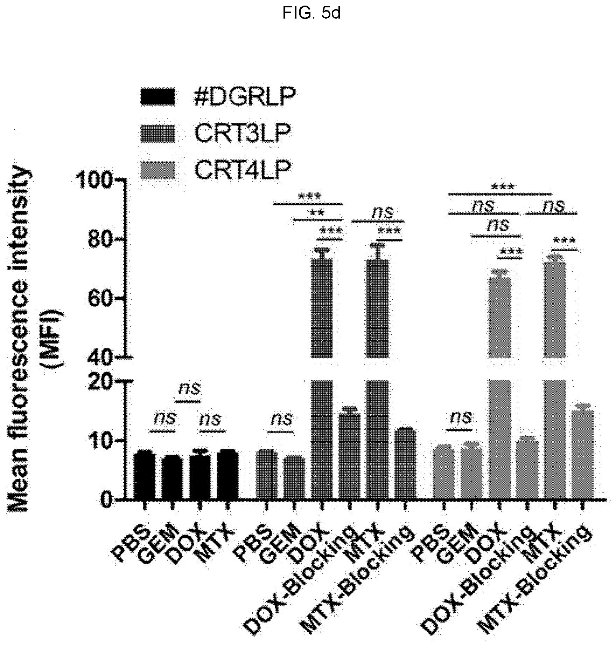

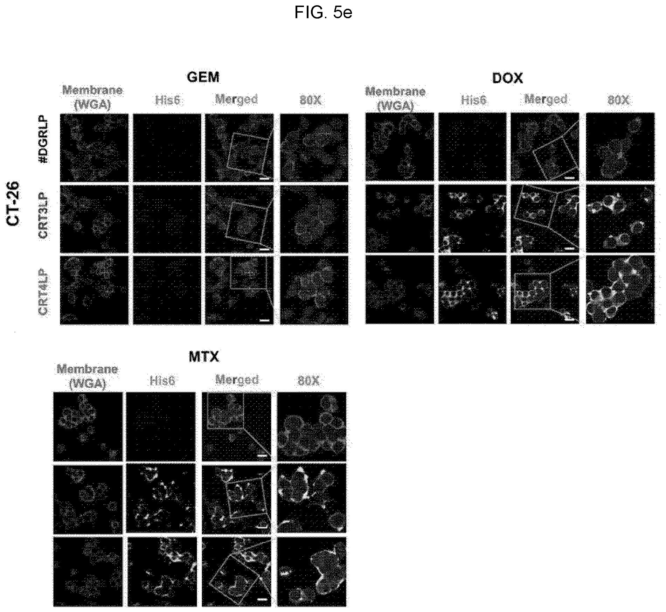

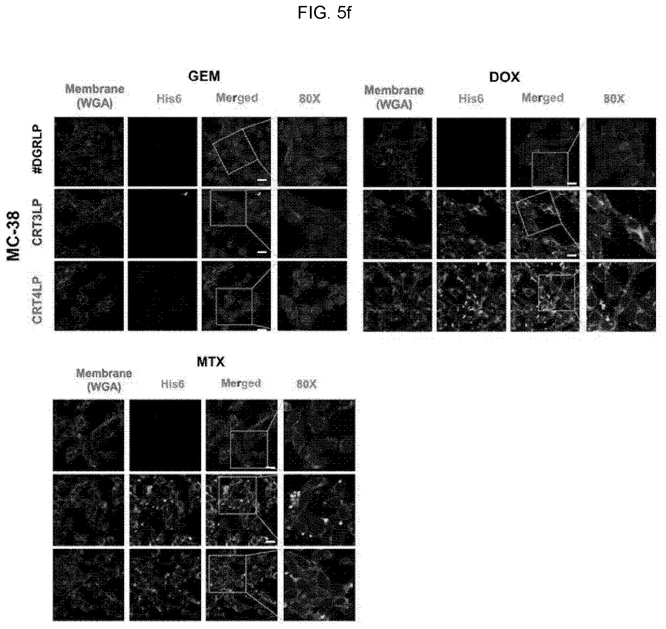

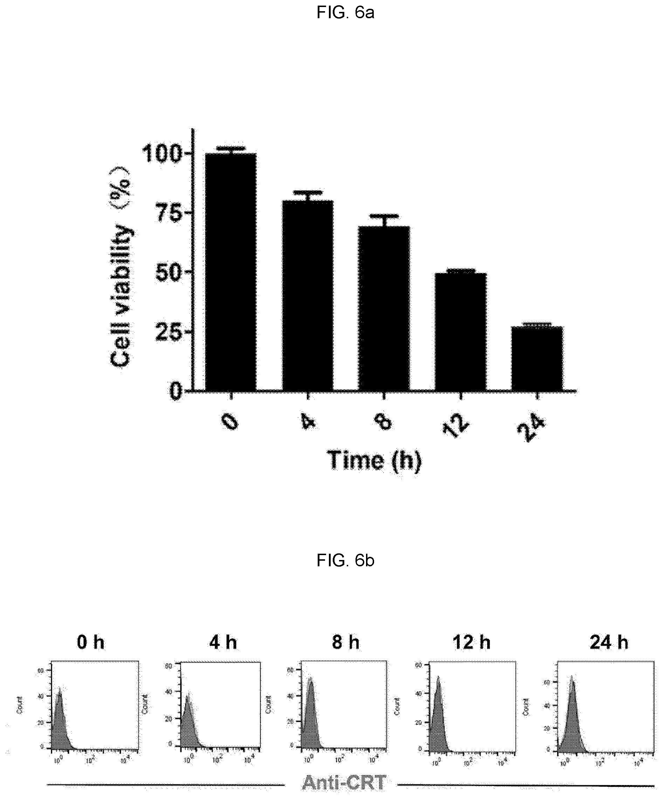

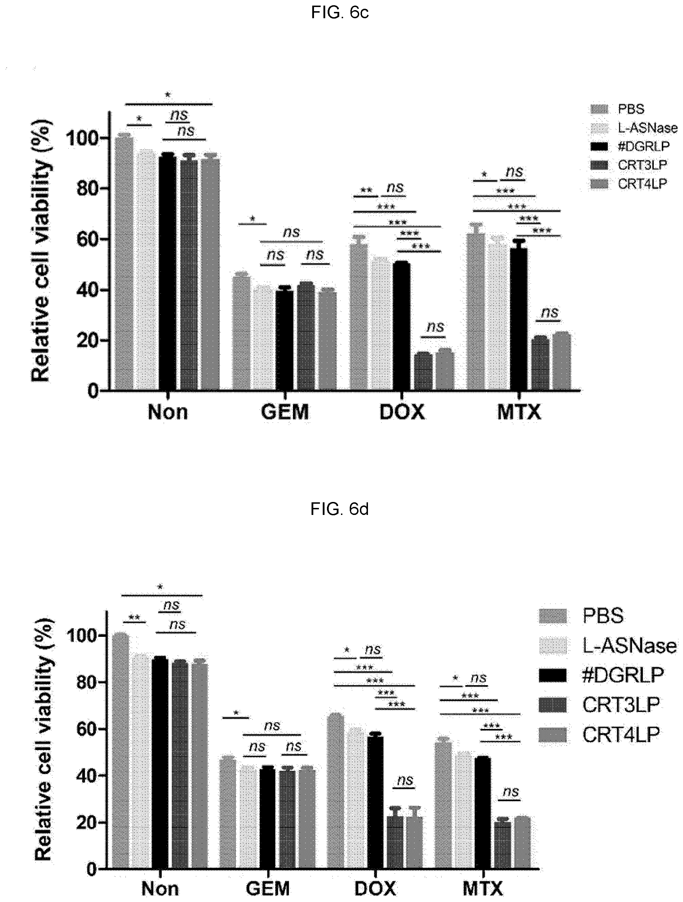

CRT3LP and CRT4LP specifically target ecto-CRT and enhance cytotoxicity against tumor cells treated with ICD-induced drugs. The inventors evaluated the specific binding of CRT3LP and CRT4LP to ecto-CRT in tumor cells treated with ICD-induced anticancer agents by flow cytometry (FIG. 5). The results showed that, similar to CRT3 and CRT4 monobodies, the proteins efficiently bound to CT-26 (FIG. 5a and 5c) and MC-38 (FIG. 5b and 5d) cells treated with DOX and MTX, but not with GEM or PBS treatment. The negative control #DGRLP did not bind to cells even in the presence of DOX and MTX. Furthermore, binding of CRT3LP and CRT4LP was significantly inhibited by pretreatment with anti-CRT antibodies in cells treated with DOX or MTX. This indicates that CRT3LP and CRT4LP specifically bind to ecto-CRTs exposed by ICD-induced anticancer agents. The above flow cytometry results were consistent with the results of immunofluorescence staining of the same cells (FIGs. 5e and 5f). CRT3LP and CRT4LP only bound to CT-26 and MC-38 cells treated with DOX or MTX, but not GEM. The negative control #DGRLP did not bind to cells even in the presence of DOX and MTX. L-ASNase induced cell death even in cells derived from solid tumors (M. van Trimpont, et al., Cancers, 14(4): 902, 2022). To test whether the cell death was ICD, CT-26 cells (200 IU/mL) were treated with L-ASNase for 24h (FIG. 6). Cells treated with the enzyme showed a progressive decrease in viability (FIG. 6a). However, no significant CRT exposure was detected in the cells (FIG. 6b). This indicates that the enzyme caused cell death via a pathway other than ICD. Subsequently, the anticancer activity of CRT3LP and CRT4LP in cells pretreated with ICD-induced drugs was measured (Figs. 6c and 6d). The viability of CT-26 cells was barely affected in the presence of low concentrations of CRT3LP or CRT4LP (1 IU/mL) (FIG. 6c). Pretreatment with anticancer agents caused a significant decrease in cell viability (45.1% in GEM, 57.9% in DOX, 62.3% in MTX). In the presence of L-ASNase or #DGRLP, cell viability was barely reduced in cells pretreated with anticancer agents; however, CRT3LP and CRT4LP reduced the viability of cells pretreated with DOX and MTX to a 2-fold higher extent. In contrast, the reduction in cell viability was not observed by CRT3LP and CRT4LP in cells pretreated with GEM. The above results were the same in MC-38 cells (FIG. 6d). This suggests that only CRT3LP and CRT4LP exhibit efficient cytotoxicity against tumor cells pretreated with ICD-induced anticancer agents.

### Example 3: Effect of CRT3LP and CRT4LP in tumor-bearing mice treated with anticancer agents

### 3-1: CT-26 tumor-bearing mice treated with DOX

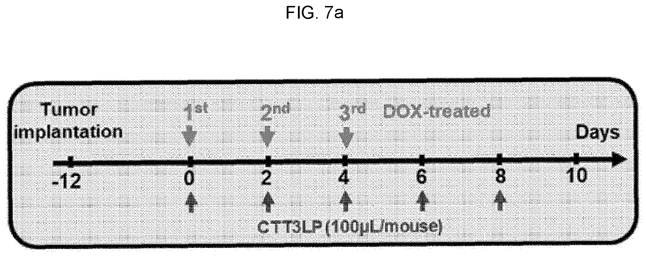

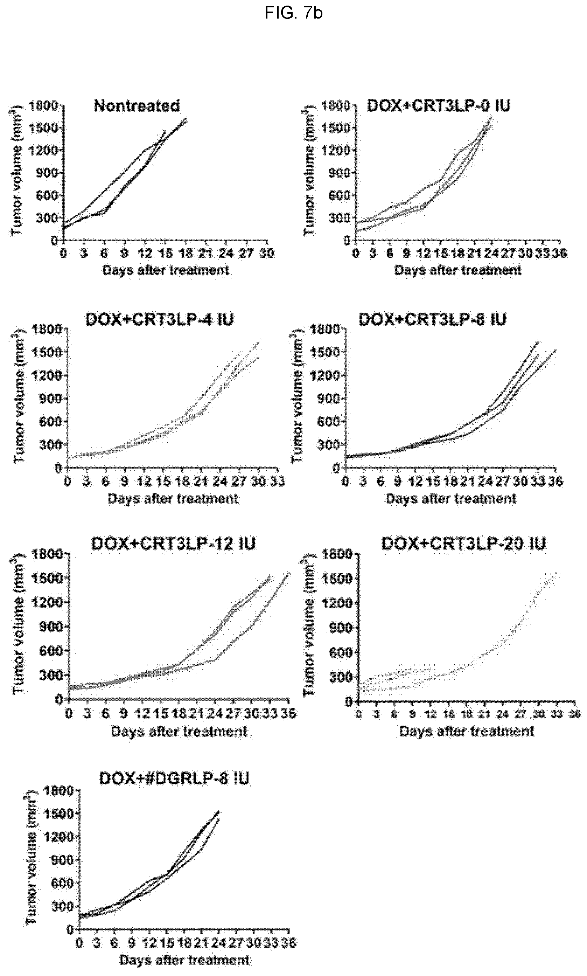

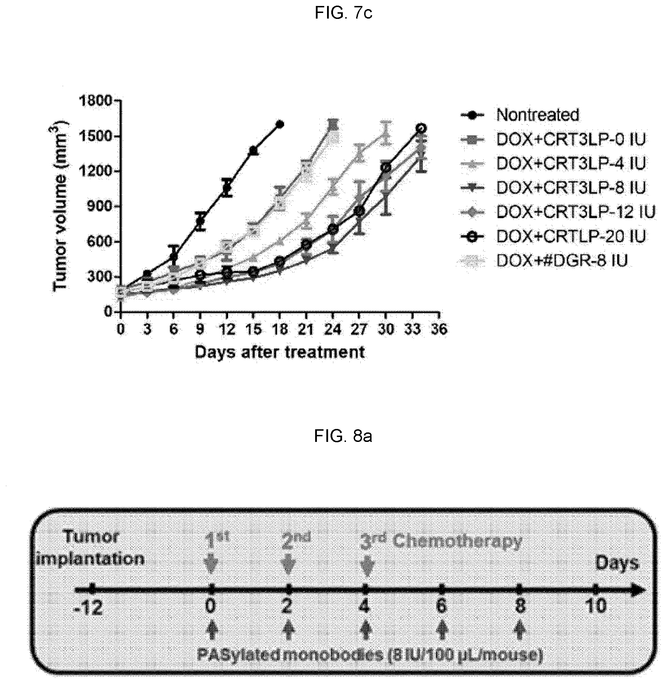

To evaluate the optimal dose of PASylated L-ASNase to exhibit anticancer effects *in vivo,* the present inventors treated CT-26 tumor-bearing BALB/c mice (n = 3 per group) with anticancer agents and CRT3LP (FIG. 7). The treatment procedure was performed as shown in FIG. 7a. First, various doses of CRT3LP (5 doses, 2 days apart) were administered intravenously to mice treated with DOX (10 mg DOX/kg/peritoneal injection, 3 doses, 2 days apart) (FIG. 7b). As a result, tumors in the DOX-treated group grew more slowly than untreated tumors. Tumor growth was inhibited to a greater extent in mice treated with DOX + CRT3LP than in mice treated with DOX alone. Tumor growth inhibition in mice treated with 8 IU of CRT3LP was similar to that of mice treated with 12 IU and better than that of mice treated with 4 IU. Notably, 20 IU of CRT3LP treatment caused sudden death in some mice by day 12 (2 out of 3 mice). Based on the above results, the present inventors concluded that the optimal therapeutic dose of CRT3LP is 8 IU/injection/mouse (FIG. 7c).

### 3-2: Combination effect of PASylated CRT-targeted L-ASNase in CT-26 tumor models

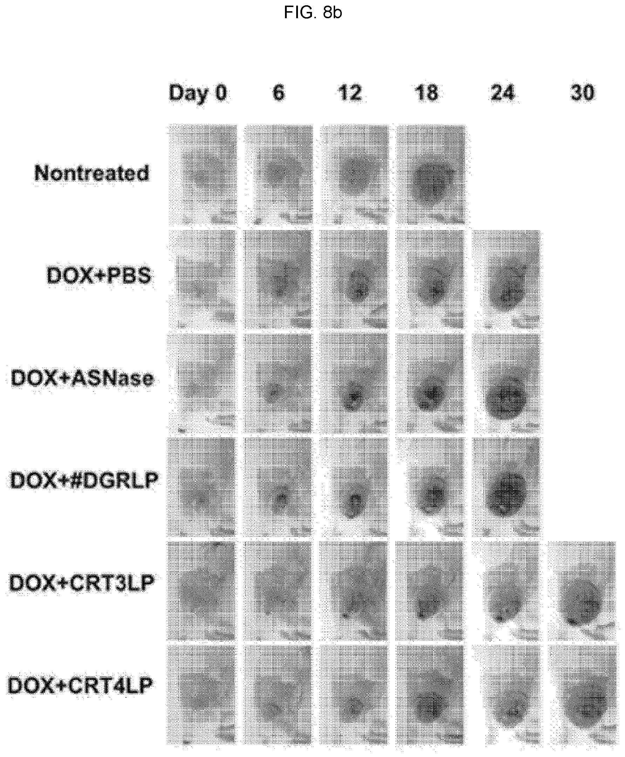

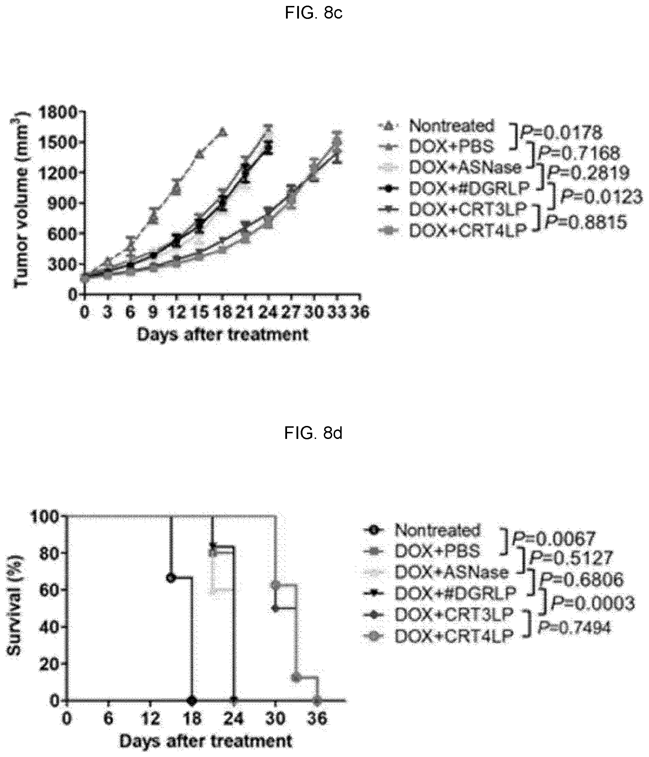

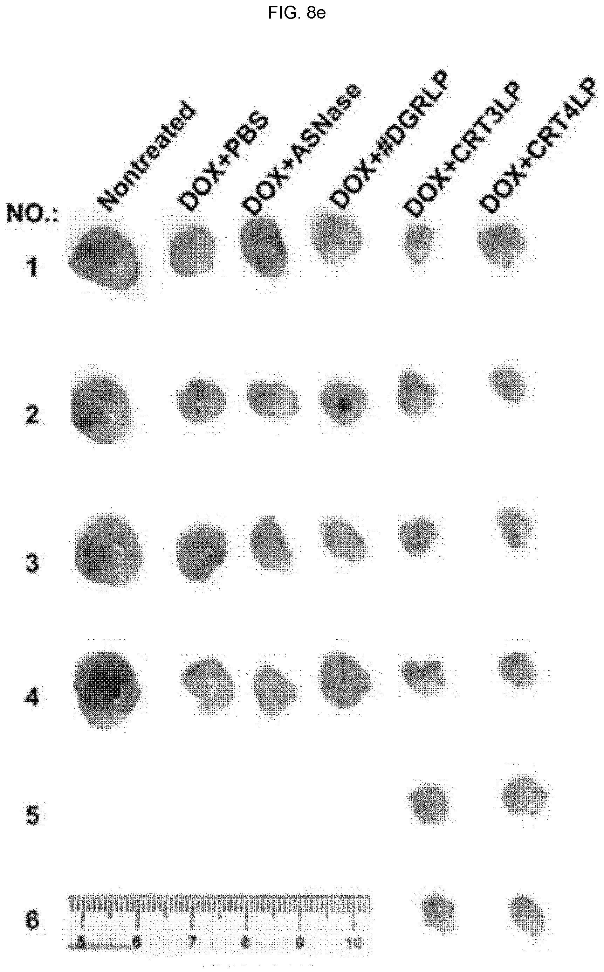

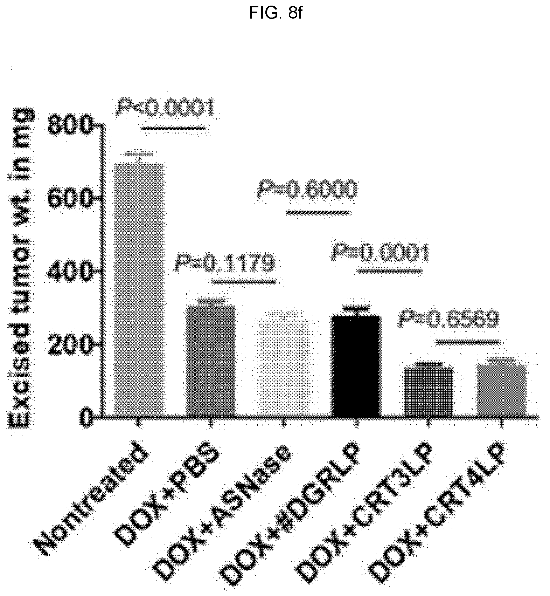

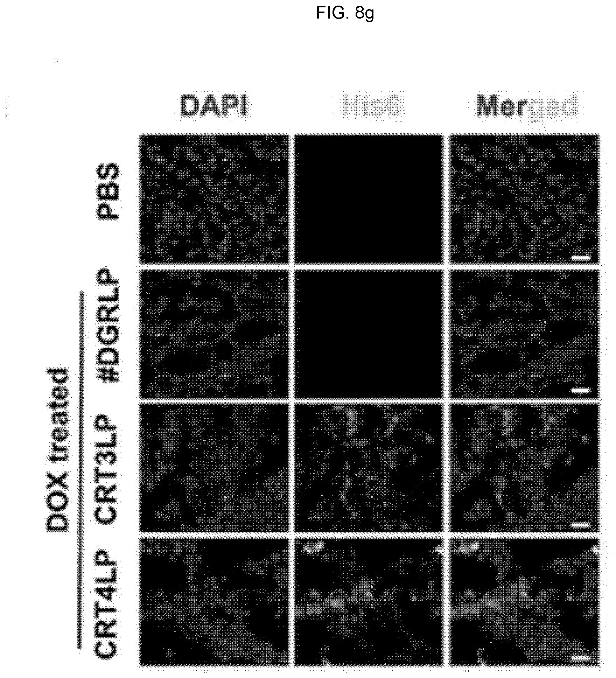

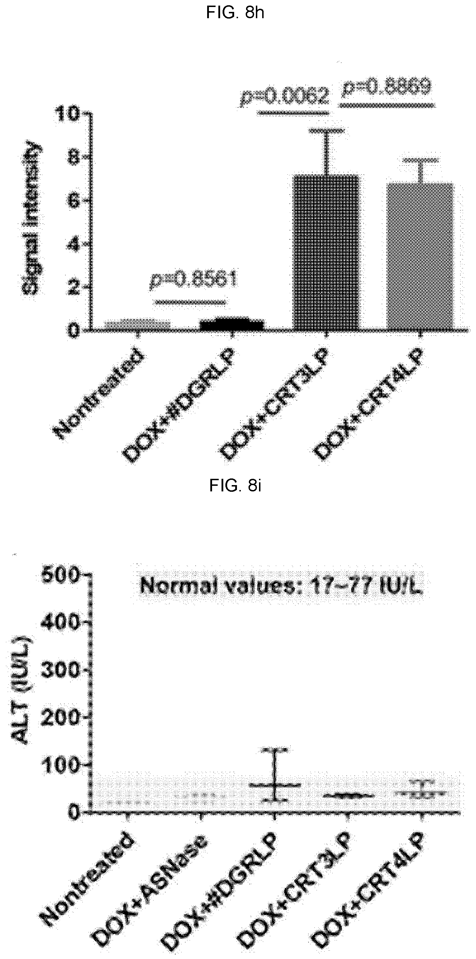

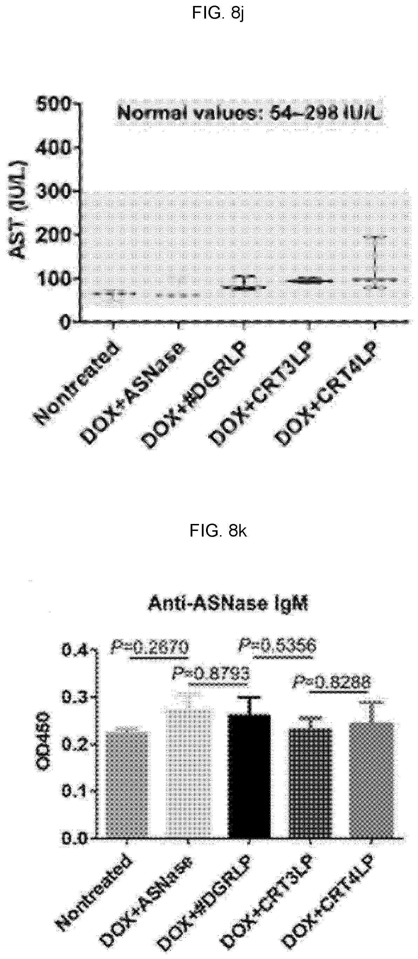

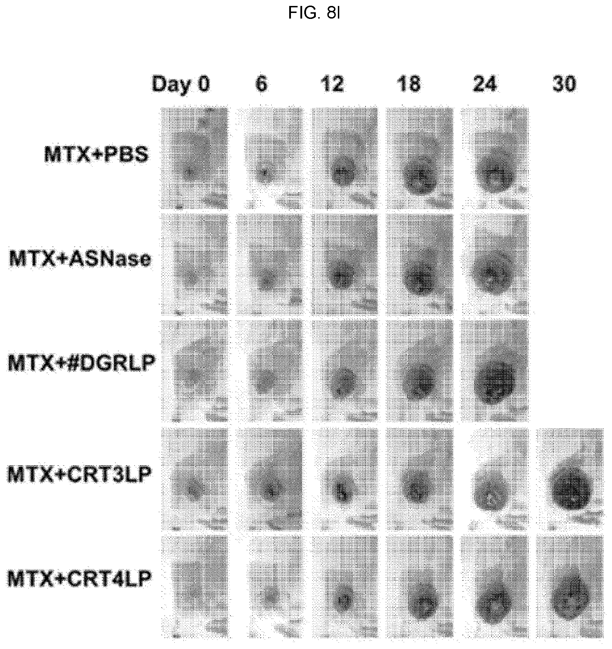

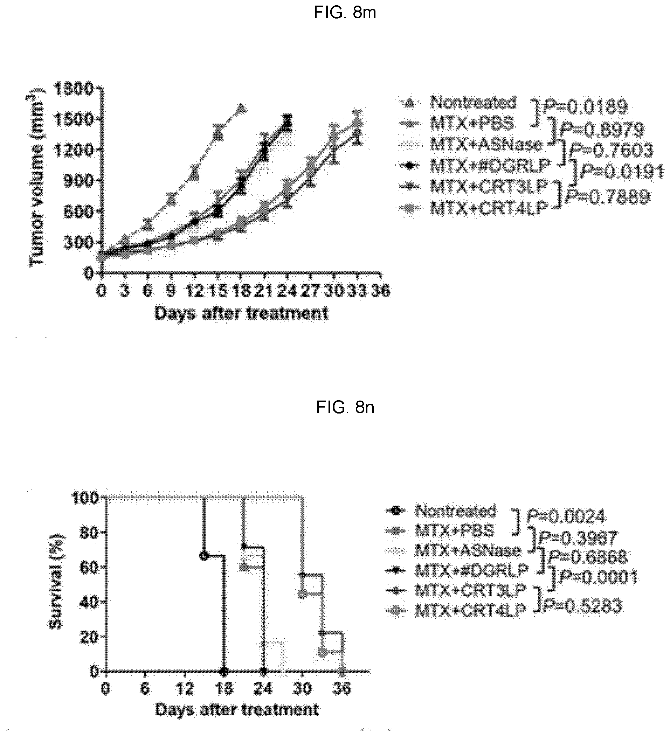

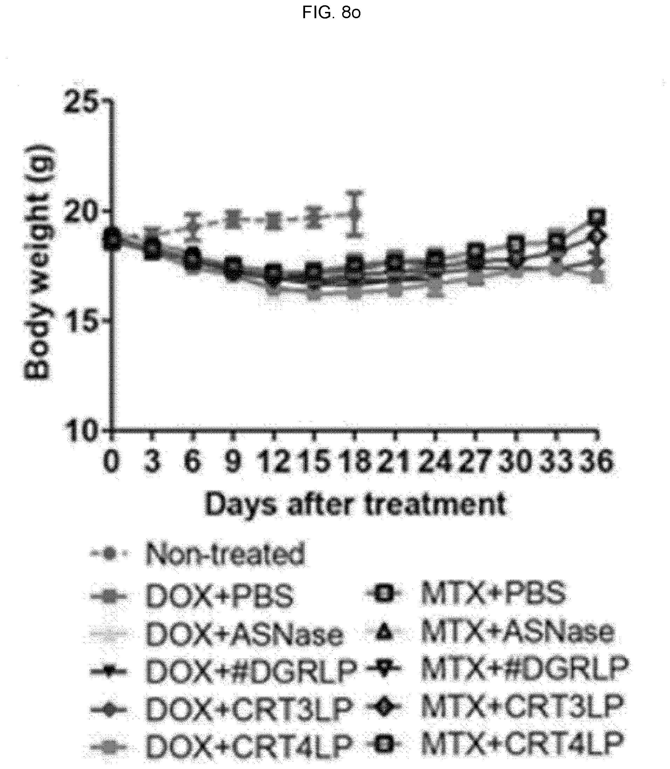

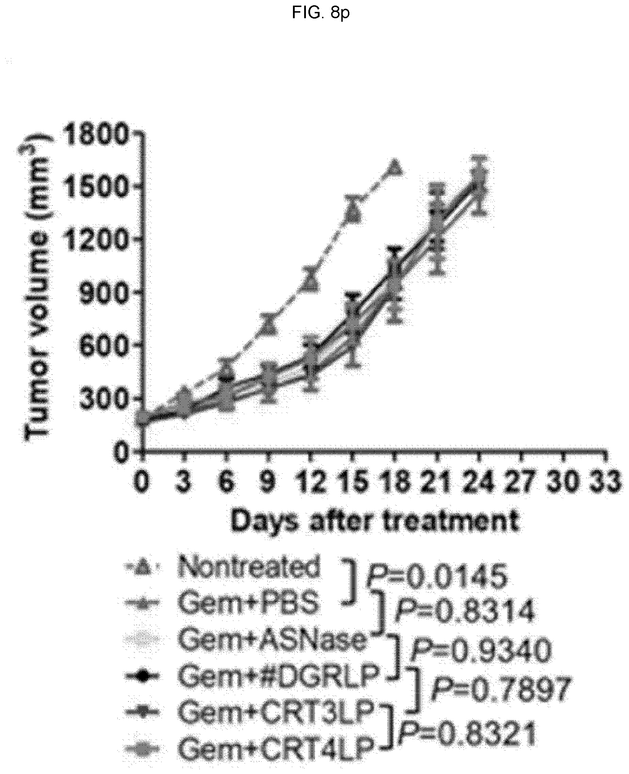

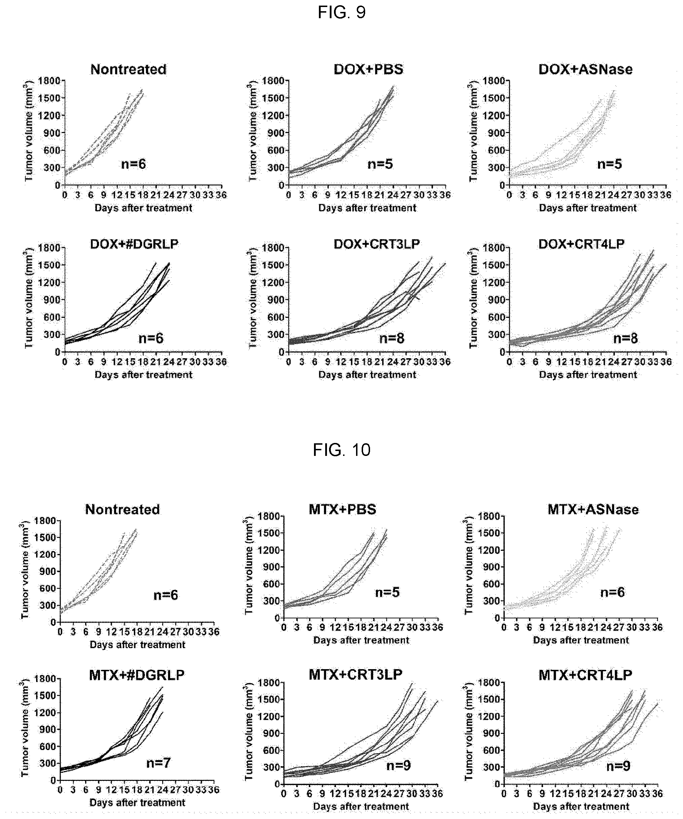

The present inventors investigated the therapeutic efficacy of an optimal therapeutic dose of PASylated CRT-targeted L-ASNase (8 IU/mouse) in combination with DOX in CT-26 tumor-bearing mice (FIG. 8). Treatment of mice was performed according to the described schedule (FIG. 8A). The results showed that DOX + L-ASNase and DOX + #DGRLP inhibited tumor growth better than no treatment, but the level of inhibition was similar to that observed after treatment with DOX alone (DOX + PBS). This indicates that the enzymatic activity of L-ASNase and #DGRLP does not increase the anti-cancer effect of DOX. DOX + CRT3LP and DOX + CRT4LP inhibited tumor growth to a higher degree than DOX + L-ASNase and DOX + #DGRLP (FIG. 8b, 8c, and FIG. 9). The survival time of mice in the DOX + CRT3LP and DOX + CRT4LP groups was prolonged by more than 1 week compared to the DOX + L-ASNase and DOX + #DGRLP groups, but the tumors were not completely eliminated (FIG. 8d). The above results were also confirmed by tumor weights measured on day 12 (FIGs. 8e and 8f). The average tumor weight was slightly lower in the DOX + L-ASNase and DOX + #DGRLP groups than in the DOX alone group (304.8 mg in DOX + PBS, 263.3 mg in DOX + L-ASNase, and 278 mg in DOX + L-ASNase). Body weight was least in the DOX + CRT3LP and DOX + CRT4LP groups (136.2 mg in DOX + CRT3LP and 143.3 mg in DOX + CRT4LP). All results were consistent with tumor growth rate and survival measurements. The targeting of CRT3LP and CRT4LP to DOX-treated CT-26 tumors was observed by confocal microscopy on day 6 (FIGs. 8g and 8h). In tumor tissues stained with anti-His tag antibody, fluorescent signals were detected in the DOX + CRT3LP and DOX + CRT4LP groups, but not in the untreated (PBS) group and DOX + #DGRLP group. The fluorescence intensity of DOX + #DGRLP and DOX + PBS was at background levels, whereas the fluorescence intensity of DOX + CRT3LP and DOX + CRT4LP was significantly increased (7.1-fold higher in DOX+CRT3LP and 6.7-fold higher in DOX+CRT4LP). The above results suggest that CRT3LP and CRT4LP specifically target ecto-CRT in DOX-treated tumor cells in vivo.

Finally, biochemical analysis of serum samples was performed on day 12 (FIGS. 8i to 8k). The levels of Ala aminotransferase (ALT) and Asp transaminase (AST), which are indicators of liver function, were increased by the high dose of DOX treatment (Y. Sadzuka, et al., nternational journal of pharmaceutics, 432(1-2): 42-49, 2012). The above levels showed little increase in all treated groups compared to the untreated group in the present invention (FIGs. 8i and 8j). These data indicate that the doses of DOX- and PASylated CRT-targeted L-ASNase of the present invention are within the clinical range. Furthermore, IgM levels against L-ASNase in serum were measured to assess the primary antibody response to CRT3LP and CRT4LP (FIG. 8k). No significant changes in levels were detected in any of the groups. This indicates that PASylated CRT-targeted L-ASNase did not induce an antibody response at the early time point of treatment combined with DOX. The therapeutic efficacy of PASylated CRT-targeted L-ASNase (8 IU/mouse) combined with MTX was measured in CT-26 tumor-bearing mice. Treatment was performed with MTX (2 mg/kg/injection, 3 times and 2 days apart) on the same schedule described in FIG. 6a. As a result, the effects of CRT3LP and CRT4LP in MTX-treated mice were similar to those in DOX-treated mice (FIGs. 8l to 8n and FIG. 10). Significant tumor inhibitory effects and prolonged survival were observed in MTX + CRT3LP and MTX + CRT4LP mice compared to MTX + L-ASNase and MTX + #DGRLP mice.

Furthermore, the weight changes of the mice were measured after 36 days of combination therapy (FIG. 8o). Body weight decreased slightly after treatment in all groups and gradually recovered. It has been reported that body weight decreased with anticancer drug treatment (K. Takayoshi, et al., Nutrition and cancer 69(3): 408-415, 2017). Therefore, the weight loss in the combination therapy was probably caused by the anticancer agents and not by the PASylated CRT-targeted L-ASNase. The therapeutic effects of CRT3LP and CRT4LP were also measured in mice treated with GEM, an anticancer drug that does not cause ICD. CRT3LP and CRT4LP did not enhance tumor suppression in mice treated with this drug (FIG. 8p). These results suggest that CRT3LP and CRT4LP exert additive anticancer effects in CT-26 tumor-bearing mice treated with ICD-induced anticancer agents.

### 3-3: Combination effect with DOX and PASylated CRT-targeted L-ASNase in MC-38 tumor models

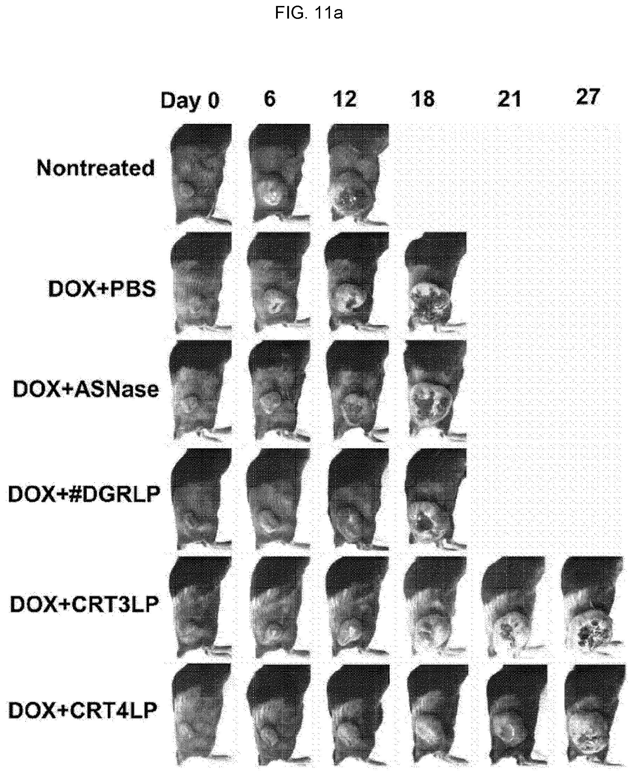

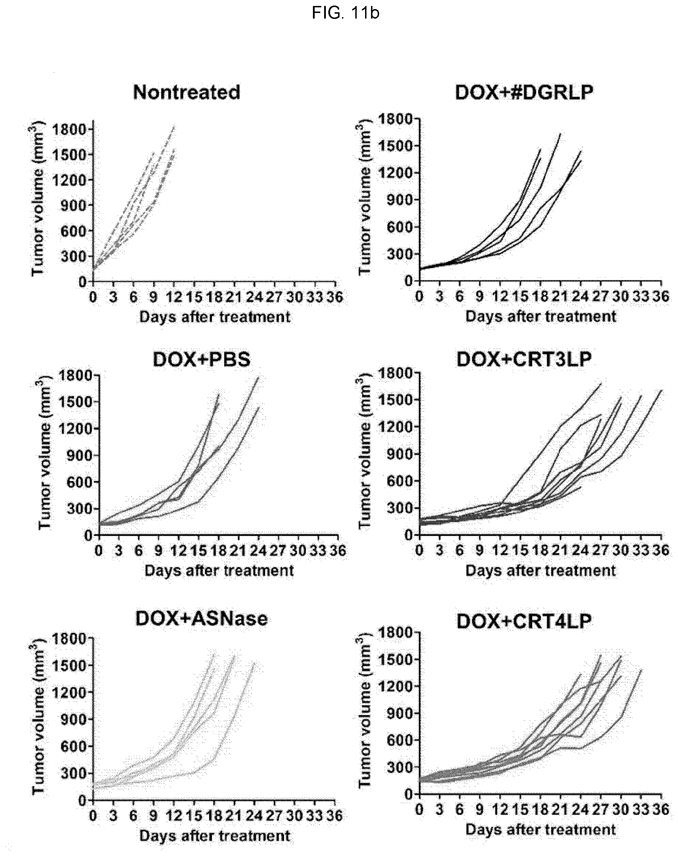

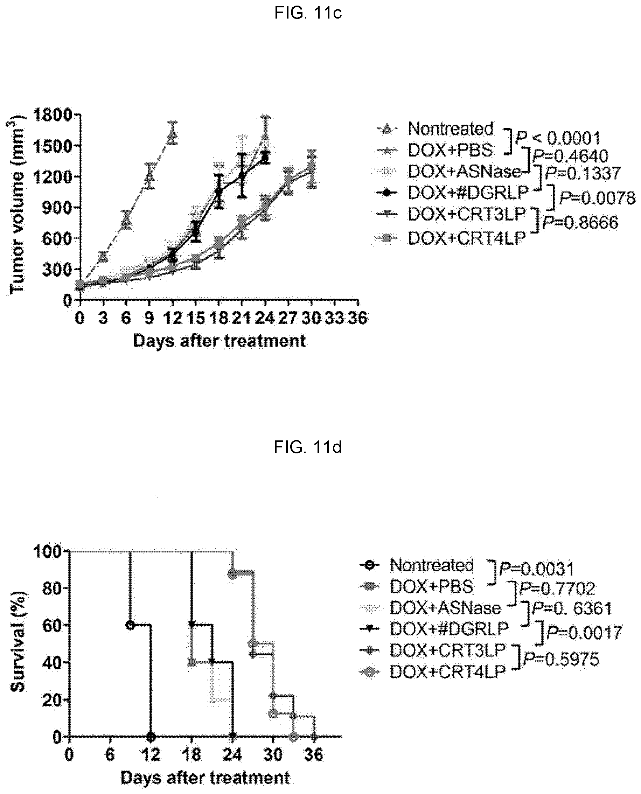

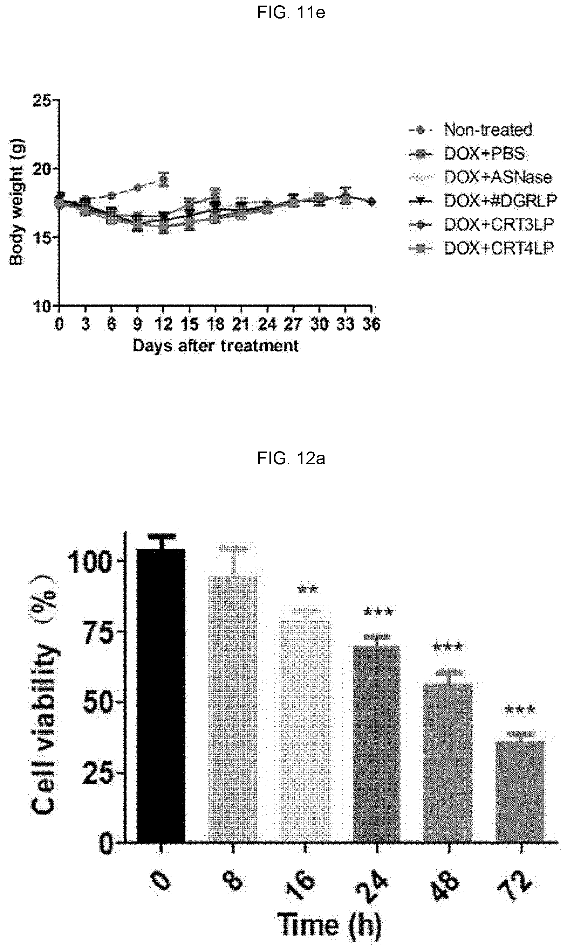

Finally, the present inventors evaluated the therapeutic efficacy of CRT3LP and CRT4LP in MC-38 tumor-bearing C57BL/6 mice treated with DOX (FIG. 11). All treatments were performed with the same procedure as used in CT-26 tumor-bearing mice. Tumor growth inhibition and increased survival were observed in the DOX + CRT3LP and DOX + CRT4LP groups compared to the other groups. The results of body weight changes were similar to those observed in CT-26 tumor-bearing mice. In conclusion, the above results suggest that PASylated CRT-targeted L-ASNase, CRT3LP and CRT4LP enhanced the efficacy of ICD-induced anticancer agents against solid tumors.

### Example 4: Combined effect of irradiation and CRT-targeted L-ASNase

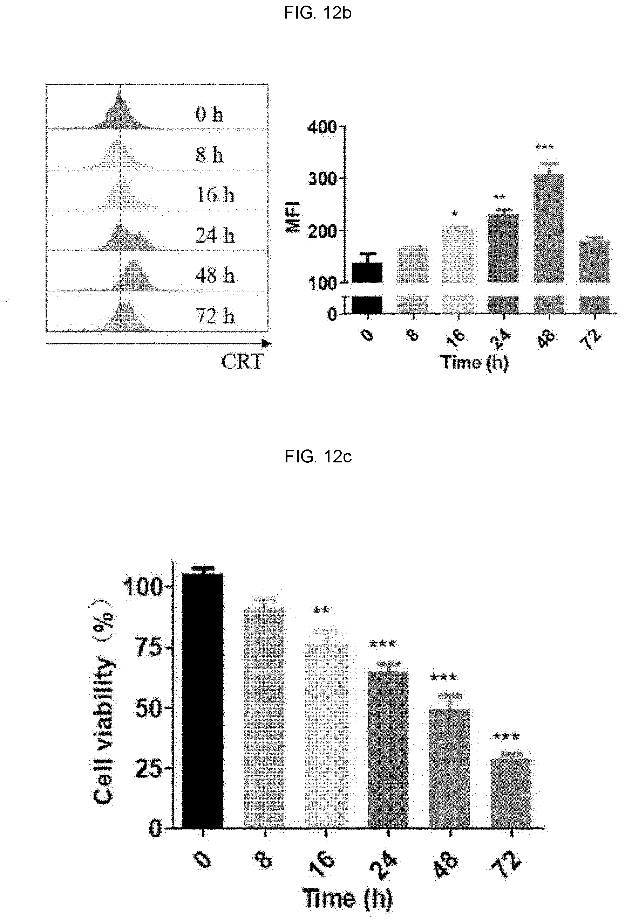

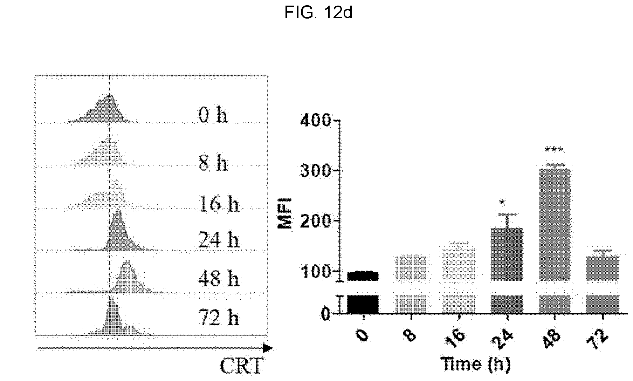

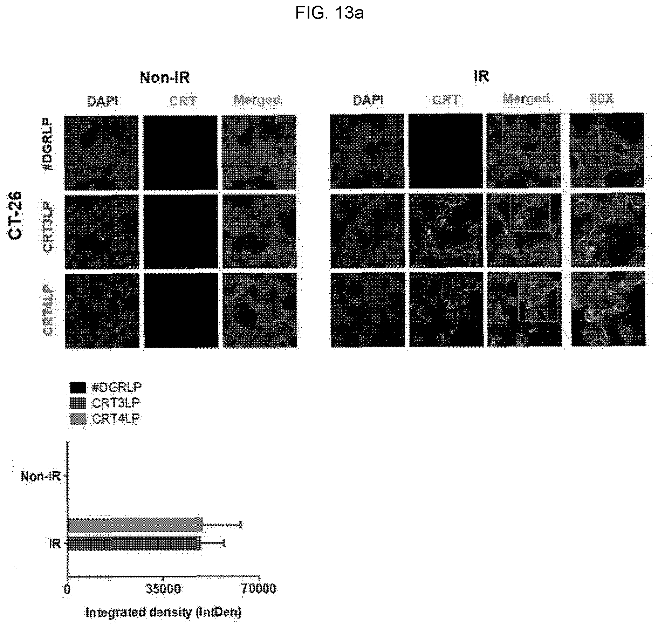

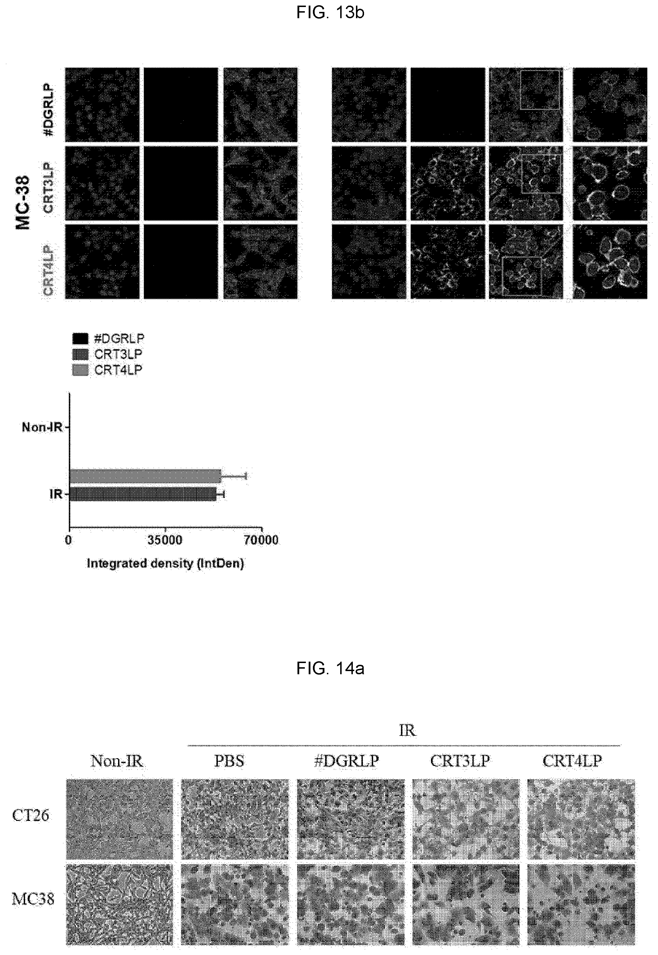

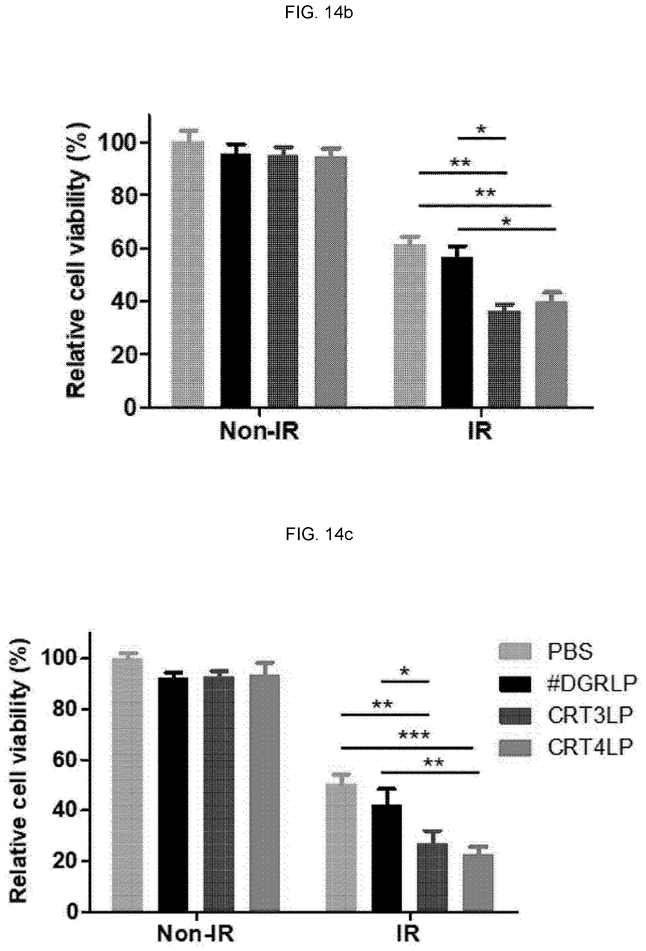

The present inventors investigated the combined effects of treatment of CT-26 and MC-38 tumor cells with the fusion proteins of the present invention after irradiation. First, CT-26 and MC-38 cells (5×10⁵) were irradiated (10 Gy, 3.3 Gy/min) for the determination of cytotoxicity and ecto-CRT translocation after irradiation induction, and the viability and CRT exposure were analyzed. It was found that CT-26 and MC-38 cells exhibited cytotoxicity, and ecto-CRTs were detected according to the specified time after irradiation treatment (FIG. 12). Furthermore, to investigate the binding of CRT-targeted L-ASNase after ICD-induced irradiation of tumor cells, cells treated with 10 Gy irradiation for 48h were stained with CRT-targeted L-ASNases, anti-His antibodies and FITC-conjugated secondary antibodies, and observed by fluorescence microscopy. It was showed that the binding of CRT-targeted L-ASNase increased after irradiation (FIG. 13). Moreover, to determine the anticancer efficacy of the fusion proteins of the present invention after irradiation, CT-26 and MC-38 cells were treated with irradiation for 24h and co-incubated with CRT-targeted L-ASNase (1 IU/mL) for 24h, and then the cells were observed under a microscope to analyze the relative cell viability. It was showed that the CRT-targeted L-ASNase of the present invention enhanced the radiation-induced cytotoxicity in tumor cells (FIG. 14).

The present invention has been described with reference to the embodiments described above, but these are exemplary only, and one having ordinary skill in the art will understand that various modifications and other equally valid embodiments are possible from them. The true scope of technical protection of the invention should therefore be determined by the technical ideas of the appended claims of the patent.

## Claims

1. A fusion protein comprising a recombinant monobody which specifically binds to clareticulin and an L-asparaginase linked to the C-terminus of the recombinant monobody, wherein the recombinant monobody has a peptide that specifically binds to the calreticulin inserted into at least one of the BC loop and the FG loop of human fibronectin domain III (Fn3).

2. The fusion protein according to claim 1, wherein the recombinant monobody has a tumor antigen-specific binding peptide inserted into both of the BC loop and the FG loop.

3. The fusion protein according to claim 1, wherein the recombinant monobody comprises amino acids represented by SEQ ID NO: 9 or 10.

4. The fusion protein according to claim 1, wherein the recombinant monobody has a calreticulin-binding peptide represented by SEQ ID NO: 15 inserted into the BC loop of the human fibronectin domain III and a calreticulin-binding peptide represented by SEQ ID NO: 16 inserted into the FG loop, or, has a calreticulin-binding peptide represented by SEQ ID NO: 16 inserted into the BC loop and a calreticulin-binding peptide represented by SEQ ID NO: 15 inserted into the FG loop.

5. The fusion protein according to claim 1, further comprising a Pro-Ala-Ser (PAS) repeat.

6. The fusion protein according to claim 5, wherein the PAS repeat comprises from 20 to 500 units.

7. The fusion protein according to claim 5, wherein the PAS repeat is linked to the N-terminus or C-terminus of the fusion protein.

8. The fusion protein according to claim 1, consisting of an amino acid sequence represented by SEQ ID NO: 23 or 24.

9. A pharmaceutical composition for the treatment of solid tumors, comprising the fusion protein of any one of claims 1 to 8 as an active ingredient.

10. The pharmaceutical composition according to claim 9, used as a radiotherapy adjuvant.

11. The pharmaceutical composition according to claim 10, further comprising one or more anti-cancer compounds, tumor suppressor proteins, or anticancer proteins.

12. The pharmaceutical composition according to claim 11, wherein the anticancer compound is selected from the group consisting of an immunogenic cell death agent, an immune checkpoint inhibitor, a mitotic inhibitor, an antimetabolite, a hormonal agent, an alkylating agent, and a topoisomerase inhibitor.

13. The pharmaceutical composition according to claim 12, wherein the immunogenic apoptosis inducer is an anthracycline-based anticancer agent, a taxane-based anticancer agent, an anti-EGFR antibody, a BK channel agonist, bortezomib, a cardiac glycoside, a cyclophosphamide-based anticancer agent, a GADD34/PP1 inhibitor, LV-tSMAC, Measles virus, or oxaliplatin.

14. The pharmaceutical composition according to claim 13, wherein the anthracycline anticancer agent is daunorubicin, doxorubicin, epirubicin, idarubicin, pixantrone, sabarubicin, or valrubicin.

15. The pharmaceutical composition according to claim 11, wherein the tumor suppressor protein is con HippelLindau (VHL), Adenomatous polyposis coli (APC), cluster of differentiation 95 (CD95), Suppression of tumorigenicity 5 (ST5), Yippee like 3 (YPEL3), p53, Suppression of tumorigenicity 7 (ST7), or Suppression of tumorigenicity 14 (ST14).

16. The pharmaceutical composition according to claim 11, wherein the anticancer protein is a protein toxin, an antibody specific for a cancer antigen, a fragment of the antibody, or an antiangiogenic factor.

17. The pharmaceutical composition according to claim 16, wherein the protein toxin is Botulinum toxin, Tetanus toxin, Shiga toxin, Diphtheria toxin (DT), ricin, Pseudomonas exotoxin (PE), cytolysin A (ClyA), or r-Gelonin.

18. The pharmaceutical composition according to claim 11, wherein the solid cancer is selected from the group consisting of lung cancer, stomach cancer, liver cancer, bone cancer, pancreatic cancer, gallbladder cancer, cholangiocarcinoma, skin cancer, head and neck cancer, cutaneous melanoma, uterine cancer, ovarian cancer, rectal cancer, colon cancer, colorectal cancer, breast cancer, uterine sarcoma, fallopian tube carcinoma, endometrial carcinoma, cervical carcinoma, vaginal carcinoma, vulvar carcinoma, esophageal cancer, laryngeal cancer, small bowel cancer, and thyroid cancer.

19. A method of treating an individual with a solid tumor, comprising administering to the individual a therapeutically effective amount of the pharmaceutical composition of any one of claims 9 to 18.

20. A method of treating an individual with a solid tumor, comprising administering to the individual a therapeutically effective amount of the fusion protein of any one of claims 1 to 8 or the pharmaceutical composition of any one of claims 9 to 18, simultaneously with or before or after irradiation.
